# EUROPÄISCHE PATENTANMELDUNG

(11) **EP 4 047 073 A1**
(43) Veröffentlichungstag der Anmeldung: **24.08.2022**
(21) Anmeldenummer: 21157529.5
(22) Anmeldetag: 17.02.2021
(51) Int. Cl.: C10B 53/07, C08J 11/12

(54) **PYROLYSE VON POLYCARBONAT-HALTIGEM MATERIAL ZUR WIEDERGEWINNUNG VON ROHSTOFFEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Es wird ein Verfahren zur Pyrolyse von Polycarbonat-haltigem Material zur Wiedergewinnung von Rohstoffen beschrieben, umfassend mindestens die folgenden Schritte
(a) Einbringung von Pyrolysegut, mindestens umfassend Material, enthaltend eine Mischung von Polycarbonat enthaltender Verbindung und Polystyrol enthaltender Verbindung, in einen Reaktor;
(b) Zersetzung zumindest des in Schritt (a) eingebrachten Materials des Pyrolyseguts in dem Reaktor bei einer Temperatur von 300°C bis 700°C unter Erhalt von in der Gasphase befindlichem Produkt als Pyrolysat und von nicht in der Gasphase befindlichem Pyrolyserückstand, wobei
(i) während der besagten Zersetzung die Menge an Sauerstoffgas im Reaktor höchstens 2,0 Vol.% bezogen auf das Gesamtvolumen der im Reaktor befindlichen Gase beträgt, und
(ii) während der besagten Zersetzung das Pyrolysat aus dem Reaktor herausgeführt wird, und
(iii) der besagte Pyrolyserückstand aus dem Reaktor herausgeführt wird;

(c) Abkühlung des herausgeführten Pyrolysates auf eine Temperatur von weniger als 300°C unter Erhalt eines von Pyrolyseprodukt, ausgewählt aus Pyrolysatkondensat, Pyrolysatresublimat oder einer Mischung daraus;
(d) optional Aufarbeitung des Pyrolyseprodukts. Unter Ausführung dieses Verfahrens in einer dafür ausgestalteten Vorrichtung lassen sich Rohstoffe zur Herstellung von Polycarbonat-haltigem Verbundmaterial, insbesondere Bisphenol-A und Styrol, aus diesen Verbundmaterialien zurückgewinnen.

## Beschreibung

Polycarbonat-haltiges Material - wie Polycarbonat Mischungen z.B. Polycarbonatharz oder Polycarbonat Verbundharz - findet als Werkstoff für Verbrauchsgüter Anwendung. Entsprechende Polycarbonatharze oder Polycarbonat Verbundharze werden durch Mischen des Polycarbonats mit anderen Polymeren hergestellt, wie z.B. durch Mischen von Polycarbonat mit Acrylnitril-Butadien-Styrol (ABS). Da diese Polycarbonat-haltigen Materialien hervorragende Eigenschaften haben wie Schlagfestigkeit, Fließfähigkeit, Zähigkeit und Flammschutz, werden sie in einer Vielzahl von Anwendungen eingesetzt, z.B. Elektrogeräten und Autos.

Nachdem obige Polycarbonatharze und Polycarbonat Verbundharze als Bestandteil vieler Handelsprodukte auf den Markt gebracht wurden, fallen sie am Ende der Lebensdauer der Handelsprodukte im Abfall als Wertstoff an. Überwiegend werden alte Handelsprodukte der Entsorgung zugeführt und gegen neue Handelsprodukte ausgetauscht. Die Gesamtmenge der dabei entstehenden Kunststoffabfälle nimmt jedes Jahr zu. Etwa 60% der Gesamtmenge dieser Abfälle wird durch Verbrennung oder auf der Deponie entsorgt.

Bei der Verbrennung des Kunststoffabfalls wird CO₂ in die Luft emittiert, das zu der globalen Erwärmung beiträgt. Plastikabfall auf den Deponien nimmt wegen seiner geringen Dichte ein großes Volumen ein und kann von dort auch zur allgemeinen Verschmutzung in Flüssen und Meeren beitragen. Aus diesem Grunde ist es wichtig, effiziente Recyclingmethoden zu entwickeln, mit denen das Abfallproblem gelöst werden kann und auch gleichzeitig fossile Ressourcen eingespart werden können.

Da der Anteil der oben genannten Polycarbonat(verbund)harze ca. 8 % des Polymermarktes ausmacht, stand das Recycling von Polycarbonaten bisher nicht im Fokus der Entwicklungen. Für die Zukunft ist es jedoch aufgrund des weiterhin wachsenden Kunststoffmarktes wichtig, für alle Polymerklassen Recycling Technologien zu entwickeln, um sowohl die CO₂ Emission zu vermindern als auch fossile Energien einzusparen.

Die Verfahren für das Recycling von Kunststoffabfällen lassen sind grob in 3 Kategorien unterteilen:
(1) mechanisches Recycling: Hierbei werden die Kunststoffabfälle so wie sie sind wieder eingeschmolzen und können dann wiederverwendet werden. Dies ist beispielsweise für sehr reine PC Abfälle möglich.
(2) chemisches und thermochemisches Recycling: Hierbei werden die Kunststoffabfälle in Monomere depolymerisiert oder zu kleineren Molekülen zersetzt, um nützliche chemische Rohstoffe zu gewinnen und
(3) thermisches Recycling, wobei die Kunststoffabfälle hierin zu Abgas und Wärmeenergie umgesetzt werden.

Die aus dem thermochemischen Recycling gewonnenen chemischen Rohstoffe können zur Synthese neuer Kunstharze oder anderer chemischer Produkte verwertet werden.

Das thermochemische Recycling wird als Pyrolyse bezeichnet. In den meisten Fällen wird die Pyrolyse für Verpackungsmüll angewendet und daraus ein Pyrolyse-Öl gewonnen, das als eine Art recyceltes Naphtha in den bekannten Raffinerie Prozessen mit Cracker als Drop in Lösung verwendet wird. Über die Pyrolyse von Polycarbonat in diesem Pyrolyseprozess ist wenig bekannt.

Die Verwendung von Katalysatoren oder Additiven kann im Pyrolyse Prozess zur Erniedrigung der Betriebstemperatur, zur Verkürzung der Reaktionszeit, zur Erhöhung der Degradationseffizienz und zur Einschränkung der Produktverteilung führen, was den Prozess effizienter macht.

Durch Einsatz von Metallhalogenid-Salzen als Katalysator, insbesondere Kupfer- und Eisenchlorid Katalysatoren, gelang es, bei der Pyrolyse von Polycarbonat bei 600° C mehr Phenolverbindungen zu erzeugen als ohne Katalysator (M. Blazsó, J. Anal. Appl. Pyrolysis, 1999, 51, 73-88). Weiter wurde die katalytische Pyrolyse von Polycarbonat durch Einsatz verschiedener Metallchlorid Verbindungen mit dem Ziel optimiert, die Verkohlungsrückstände zu reduzieren (J. Chiu et al., Waste Manage., 2006, 26, 252-259). Der Einfluss des Katalysators wurde bei 400 °C (1 h) bestimmt, da bei dieser Temperatur die nicht-katalysierte Pyrolyse sehr langsam abläuft. Die Wahl des Metallchlorids hatte einen großen Effekt auf die Pyrolyse. NaCl, CrCl₃, CuCl₃ und AlCl₃ verbesserten die Pyrolyseraten nicht, während SnCl₂ und ZnCl₂ das Polycarbonat mit einem Gewichtsverlust von über 80% abbauten.

Durch die Verwendung von Metallsalz-Katalysatoren ließ sich ebenfalls die Produktverteilung der Pyrolyse von Polycarbonat verbessern. Während in der nicht katalytischen Polycarbonat-Pyrolyse das flüssige Produkt elf verschiedene Produkte enthielt, erhielten die Gemische mit ZnCl₂ oder SnCl₂ als Katalysator nur sieben Hauptprodukte, einschließlich Bisphenol A, Phenol und Diphenylether mit geringeren Mengen an Verunreinigungen.

Für die Pyrolyse von Polycarbonat-haltigem Material sind auch Katalysatoren bestehend aus Metalloxiden und Zeolithen mit unterschiedlichen Porösitäten und Säure/Basizität bekannt (E. V. Antonakou et al. Polym. Degrad. Stab., 2014, 110, 482-491. M. N. Siddiqui et al., Thermochim. Acta, 2019, 675, 69-76).

Mit Hilfe basischer Katalysatoren, wie CaO und MgO, ließ sich in der Pyrolyse signifikant die Temperatur auf 400-450 ° C senken, bei gleichzeitiger Produktion flüssiger Fraktionen mit hohen Anteilen an phenolischen Verbindungen (D. S. Achilias et al., J. Appl. Polym. Sci., 2009, 114, 212-221. E. C. Vouvoudi et al., Front. Environ. Sci. Eng., 2017, 11, 9). Aus einer Mischung von Polypropylen (PP), Acrylnitril-Butadien-Styrol (ABS), High Impact Polystyrol (HIPS) und Polycarbonat konnten eine langsame Freisetzung von BPA und CO₂ aus Polycarbonat bei 440 ° C nachgewiesen werden.

Unter Einfluss von Erdalkalioxiden und Hydroxiden (MgO und Mg(OH)₂) konnte in der Dampf Pyrolyse von reinem Polycarbonat eine bessere Effizienz und höhere BPA-Ausbeute (78%) bei 300 ° C erreicht werden, während beim Erhitzen auf 500 ° C mit Hilfe von MgO ein großer Anteil an Phenol (84%) hergestellt wurde (T. Yoshioka et al.,Polym. Degrad. Stab., 2009, 94, 1119-1124, T. Yoshioka et al., Ind. Eng. Chem. Res., 2014, 53, 4215-4223).

In der Druckschrift US 2007/0185309 A1 wird eine Methode zur Depolymerisation von Polycarbonaten mit Wasser in superkritischen oder subkritischen Zustand beschrieben. Eine hochreine Dihydroxy-Komponente (BPA,) die ein Bestandteil des Polycarbonats ist, konnte in hoher Ausbeute mit dieser Methode erhalten werden. Diese Depolymerisationsmethode ist umweltfreundlich, weil sie auf die Nutzung von organischen Lösemitteln verzichtet. Sie zeigt eine hohe Zersetzungsrate und wenig Nebenprodukte.

Die im Stand der Technik beschriebenen Pyrolyse-Verfahren eignen sich allesamt nicht für die industrielle bzw. gewerbliche Anwendung, da sie zumeist als Pyrolysen im Microgramm Maßstab für analytische Zecke vorgesehen sind. Die im Stand der Technik dargelegten Pyrolysen werden daher jeweils in nicht für die industrielle bzw. gewerbliche Anwendung geeigneten Pyrolysevorrichtungen ausgeführt.

Insbesondere für die Pyrolyse größerer Mengen an Polycarbonat-haltigen Materialien, die neben Polycarbonat zusätzlich weitere Inhaltsstoffe umfassen, sind neue Verfahren mit passenden Reaktoren nötig, mit deren Hilfe sich der Einfluss der weiteren Inhaltsstoffe auf das Ergebnis der Pyrolyse des Polycarbonats verringern lässt. Trotz der Gegenwart von weiteren Inhaltsstoffen sollen aus Polycarbonat-haltigen Polymermischungen gezielt Pyrolyseprodukte erhalten werden, die einem hohen Gehalt an für die Polycarbonat-Synthese wieder verwertbaren Spaltprodukten, insbesondere aromatische Hydroxyverbindungen wie z.B. Bisphenol-A (BPA), enthalten.

Zusätzlich soll ein besagtes neues Verfahren zur Pyrolyse ein solches Pyrolyseprodukt liefern, in dem neben den in der Polycarbonat-Synthese wiederverwertbaren Spaltprodukten auch solche Spaltprodukte der anderen Inhaltsstoffe, z.B. Styrol aus Polystyrol-haltigem Material, in signifikanter Menge und guter Ausbeute enthalten sind, die sich für die Herstellung dieser besagten Inhaltsstoffe im Sinne einer zyklischen Wiederverwertung eignen.

Es war daher die Aufgabe, für die gewerbliche Ausführung einer Pyrolyse ein Verfahren sowie darin nutzbare Pyrolysevorrichtungen für die Pyrolyse von Pyrolysegut, mindestens umfassend Material, enthaltend eine Mischung von Polycarbonat enthaltender Verbindung und Polystyrol enthaltender Verbindung bereitzustellen, mit dessen Einsatz auch bei größeren Mengen von Pyrolysegut eine Menge an Pyrolyseprodukt erhalten wird, das für die Synthese von Polycarbonat-haltigem und von Polystyrol-haltigem Material wiederverwertbare Spaltprodukte, bevorzugt in einer Menge von mehr als 40 Gew.-% bezogen auf das Gesamtgewicht an eingesetztem Polycarbonat-haltigem und Polystyrol-haltigem Material, enthält.

Ein Gegenstand der vorliegenden Anmeldung ist deshalb ein Verfahren zur Pyrolyse , umfassend mindestens die folgenden Schritte
(a) Einbringung von Pyrolysegut, mindestens umfassend Material, enthaltend eine Mischung von Polycarbonat enthaltender Verbindung und Polystyrol enthaltender Verbindung, in einen Reaktor;
(b) Zersetzung zumindest des in Schritt (a) eingebrachten Materials des Pyrolyseguts in dem Reaktor bei einer Temperatur von 300°C bis 700°C unter Erhalt von in der Gasphase befindlichem Produkt als Pyrolysat und nicht in der Gasphase befindlichem Pyrolyserückstand, wobei
   (i) während der besagten Zersetzung die Menge an Sauerstoffgas im Reaktor von 0 bis 2,0 Vol.% bezogen auf das Gesamtvolumen der im Reaktor befindlichen Gase beträgt, und
   (ii) während der besagten Zersetzung das besagte Pyrolysat aus dem Reaktor herausgeführt wird, und
   (iii) der besagte Pyrolyserückstand aus dem Reaktor herausgeführt wird;
(c) Abkühlung des herausgeführten Pyrolysates auf eine Temperatur von weniger als 300°C unter Erhalt von Pyrolyseprodukt, ausgewählt aus Pyrolysatkondensat, Pyrolysatresublimat oder einer Mischung daraus;
(d) optional Aufarbeitung des Pyrolyseproduktes.

Als "Pyrolysegut" wird die Gesamtheit der in den Reaktor zur Pyrolyse eingebrachten Stoffe bezeichnet, die dort in Abwesenheit von Sauerstoffgas oder in Gegenwart einer reduzierten Menge an Sauerstoffgas thermisch behandelt wird. Das Pyrolysegut ist vor der Einbringung in den Reaktor bevorzugt festförmig.

Unter "Pyrolysat" wird die Gesamtheit diejenigen durch Pyrolyse gebildeten Produkte verstanden, die sich unter den Bedingungen des Schrittes (b) (insbesondere als Gas und/oder als Aerosol) in der Gasphase des Reaktors befinden.

Unter "Pyrolyserückstand" wird die Gesamtheit derjenigen durch Pyrolyse gebildeten Stoffe und der sonstigen Rückstände des Pyrolyseguts verstanden, die sich unter den Bedingungen des Schrittes (b) nicht in der Gasphase des Reaktors befinden. Es sind solche Ausführungsformen des Verfahres bevorzugt, die sich dadurch kennzeichnen, dass der Pyrolyserückstand im Reaktor festförmig ist.

Unter "Pyrolyseprodukt" wird die Gesamtheit derjenigen Produkte aus dem Pyrolysat verstanden, die in Schritt (c) bei der Abkühlung des Pyrolysates durch Kondensation und/oder Resublimation anfallen. Pyrolyseprodukt, das flüssig ist, wird auch als Pyrolyseöl bezeichnet.

Falls nicht explizit im Zusammenhang anders definiert, ist ein Stoff (z.B. Material, Pyrolysegut, Pyrolysat, Pyrolyseprodukt, Pyrolyserückstand) "flüssig", wenn er bei 20°C und 1013 mbar im flüssigen Aggregatzustand vorliegt. Falls nicht explizit im Zusammenhang anders definiert, ist ein Stoff (z.B. Material, Pyrolysegut, Pyrolysat, Pyrolyseprodukt, Pyrolyserückstand) "festförmig", wenn er bei 20°C und 1013 mbar im festen Aggregatzustand vorliegt. Falls nicht explizit im Zusammenhang anders definiert, ist ein Stoff (z.B. Material, Pyrolysegut, Pyrolysat, Pyrolyserückstand) "gasförmig", wenn er bei 20°C und 1013 mbar im als Gas vorliegt.

Ein Stoff ist "organisch", wenn seine chemische Struktur mindestens eine kovalente Kohlenstoff-Wasserstoff-Bindung enthält.

Bei den im Rahmen dieser Anmeldung für Polymere bzw. polymere Inhaltsstoffe angegebenen mittleren Molmassen handelt es sich - sofern nicht explizit anders gekennzeichnet - stets um gewichtsmittlere Molmassen Mw, die grundsätzlich mittels Gelpermeationschromatographie mit Hilfe eines RI-Detektors bestimmbar sind, wobei die Messung zweckmäßig gegen einen externen Standard erfolgt.

Eine "Polycarbonat enthaltene Verbindung" ist eine polymere Verbindung, ausgewählt aus einem Homo- oder Copolymer, die durch Polyreaktion erhalten wird und wobei mindestens eine Wiederholungseinheit mindestens eine Struktureinheit 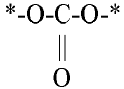 enthält, worin * eine Valenz des Polymerrückgrats kennzeichnet.

Eine "Polystyrol enthaltene Verbindung" ist eine polymere Verbindung, ausgewählt aus einem Homo- oder Copolymer, die durch Polyreaktion erhalten wird und als Wiederholungseinheit mindestens eine Struktureinheit *-CHPh-CH₂-* enthält, worin * eine Valenz des Polymerrückgrats kennzeichnet.
Ein "Reaktor" ist ein Volumen, in dem eine chemischen Umwandlung, z.B. eine thermische Zersetzung von Material aus dem Pyrolysegut, stattfindet. Dies kann für eine thermische Zersetzung beispielsweise das Volumen eines beheizten Gefäßes sein, in dem sich das Pyrolysegut befindet.

Es ist erfindungsgemäß vorteilhaft, das Pyrolysegut gemäß erfindungsgemäßem Verfahren in einen Reaktor einzubringen, der sich dadurch gekennzeichnet, dass er ausgewählt wird aus kontinuierlichem Rührkesselreaktor (CSTR), Festbettreaktor, Fließbettreaktor, Schneckenreaktor, Schneckenförderer Reaktor, Flugstromreaktor, Mitreißstromreaktor, Drehrohrreaktor, Fließbettreaktor, Schaufelreaktor. Insbesondere sind solche Reaktoren bevorzugt geeignet, in denen das Pyrolysegut kontinuierlich eingebracht werden kann und werden insbesondere ausgewählt aus Drehrohrreaktor, kontinuierlichem Rührkesselreaktor (CSTR), Festbettreaktor (insbesondere mit kontinuierlichem Bettaustausch (Schachtreaktor) mit innenliegendem Wärmetauscher, bevorzugt mit innenliegenden Wärmetauscherohren), Schneckenreaktor, Schneckenfördererreaktor, Flugstromreaktor, Drehrohrreaktor oder Wirbelschichtreaktor. Ein ganz besonders bevorzugter Reaktor einer Ausführungsform des Verfahrens wird ausgewählt aus Schneckenreaktor, Drehrohrofen oder Wirbelbett. Weitere für das erfindungsgemäße Verfahren bevorzugte Reaktoren und deren Ausführungsformen werden im Rahmen der Ausführungsformen der erfindungsgemäßen Vorrichtung zur Pyrolyse, sowie im Rahmen einer Ausführungsform des Verfahrens mit Einsatz von Katalysator, beschrieben (*vide infra*).

Das in den Reaktor eingebrachte Pyrolysegut umfasst erfindungsgemäß mindestens ein Material, enthaltend eine Mischung von Polycarbonat enthaltender Verbindung und Polystyrol enthaltender Verbindung.

Im Rahmen einer Ausführungsform des erfindungsgemäßen Verfahrens, enthält das besagte Pyrolysegut das besagte Material in einer Gesamtmenge von 10,0 bis 80,0 Gew.-%, weiter bevorzugt 30,0 bis 70,0 Gew.-%, jeweils bezogen auf das Gesamtgewicht des Pyrolysegutes.

Es hat sich als vorteilhaft erwiesen, wenn das besagte Material des Pyrolyseguts bevorzugt in Form festförmiger Teilchen (insbesondere in Form eines körnigen Gemenges) in den Reaktor eingebracht wird. Ein körniges Gemenge desbesagten Materials wird aus einer Vielzahl an losen, festförmigen Partikeln des besagten Materials gebildet, die wiederum sogenannte Körner umfassen. Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen. Die Rieselfähigkeit eines körnigen Gemenges betrifft sein Vermögen, unter eigenem Gewicht frei aus einem Rieseltesttrichter mit einem Auslauf von 16,5 mm Durchmesser zu rieseln.

Vorzugsweise weisen die festförmigen Teilchen, insbesondere die losen, festförmigen Partikel des körnige Gemenges, des in den Reaktor eingebrachten besagten Materials einen mittleren Durchmesser X_{50,3} (Volumenmittel) von 0,01 mm bis 5 cm, bevorzugt von 0,1 mm bis 5 cm, auf. Der mittlere Teilchengrößendurchmesser X_{50,3} wird durch Siebung oder mittels eines Partikelgrößenanalysators Camsizer der Fa. Retsch bestimmt.

Generell kann dieses besagte Material beispielsweise ein Homopolymer, ein Copolymer, ein Kammpolymer, ein Blockpolymer oder Mischungen davon, sein.
Es sind solche besagte Materialien besonders geeignet, in denen neben besagter Polystyrol enthaltender Verbindung als Polycarbonat enthaltende Verbindung mindestens eine Verbindung enthalten ist, die mindestens zehn
Struktureinheiten 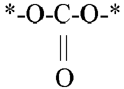 enthält, worin * eine Valenz des Polymerrückgrats kennzeichnet.

Für das erfindungsgemäße Verfahren geeignete Polycarbonat enthaltende Verbindungen für besagte Materialien sind beispielsweise aromatische Polycarbonate und/oder aromatische Polyestercarbonate. Diese sind literaturbekannt oder nach literaturbekannten Verfahren herstellbar (zur Herstellung aromatischer Polycarbonate siehe beispielsweise Schnell, "Chemistry and Physics of Polycarbonates", Interscience Publishers, 1964 sowie die DE-AS 1 495 626, DE-A 2 232 877, DE-A 2 703 376, DE-A 2 714 544, DE-A 3 000 610, DE-A 3 832 396; zur Herstellung aromatischer Polyestercarbonate, z. B. DE-A 3 007 934).

Die Herstellung der als eine Polycarbonat enthaltende Verbindung einsetzbaren aromatischen Polycarbonate erfolgt z. B. durch Umsetzung von Verbindungen mit mindestens zwei Hydroxygruppen, insbesondere von Diphenolen, mit Kohlensäurehalogeniden, vorzugsweise Phosgen und/oder mit aromatischen Dicarbonsäuredihalogeniden, vorzugsweise Benzoldicarbonsäuredihalogeniden, nach dem Phasengrenzflächenverfahren, gegebenenfalls unter Verwendung von Kettenabbrechern, beispielsweise Monophenolen und gegebenenfalls unter Verwendung von trifunktionellen oder mehr als trifunktionellen Verzweigern, beispielsweise Triphenolen oder Tetraphenolen. Ebenso ist eine Herstellung über ein Schmelzepolymerisationsverfahren durch Umsetzung von Verbindungen mit mindestens zwei Hydroxylgruppen, insbesondere von Diphenolen, mit beispielsweise Diphenylcarbonat möglich.

Erfindungsgemäß sind solche besagte Materialien vorteilhaft einsetzbar, in denen die Polycarbonat enthaltende Verbindung mindestens eine Verbindung ist, die durch Umsetzung von zumindest den Verbindungen (i) mindestens einer aromatischen Verbindung mit mindestens zwei Hydroxylgruppen, besonders bevorzugt Bisphenol-A, und (ii) Phosgen oder Diphenylcarbonat erhalten wurde.

Zur Darstellung der Polycarbonat enthaltenden Verbindung wird bevorzugt mindestens eine aromatische Verbindung mit mindestens zwei Hydroxylgruppen verwendet, die aus der allgemeinen Formel (I) ausgewählt wird, 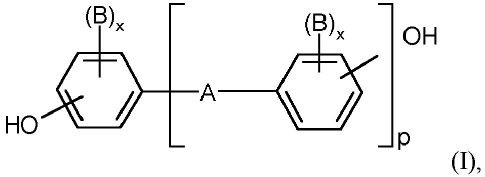 worin
A eine Einfachbindung, C₁ bis C₅-Alkylen, C₂ bis C₅-Alkyliden, C₅ bis C₆-Cycloalkyliden, - O-, -SO-, -CO-, -S-, -SO₂-, C₆ bis C₁₂-Arylen, an das weitere aromatische gegebenenfalls Heteroatome enthaltende Ringe kondensiert sein können,
   oder ein Rest der Formel (II) oder (III) 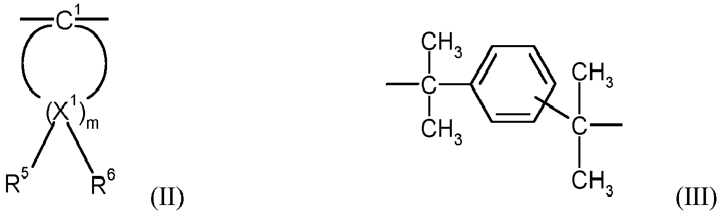
B jeweils C₁ bis C₁₂-Alkyl, vorzugsweise Methyl, Halogen, vorzugsweise Chlor und/oder Brom
x jeweils unabhängig voneinander 0, 1 oder 2,
p 1 oder 0 sind, und
R⁵ und R⁶ für jedes X¹ individuell wählbar, unabhängig voneinander Wasserstoff oder C₁ bis C₆-Alkyl, vorzugsweise Wasserstoff, Methyl oder Ethyl,
X1 Kohlenstoff und
m eine ganze Zahl von 4 bis 7, bevorzugt 4 oder 5 bedeuten, mit der Maßgabe, dass an mindestens einem Atom X¹, R⁵ und R⁶ gleichzeitig Alkyl sind.

Bevorzugte aromatische Verbindungen mit mindestens zwei Hydroxylgruppen sind Hydrochinon, Resorcin, Dihydroxydiphenole, Bis-(hydroxyphenyl)-C₁-C₅-alkane, Bis-(hydroxyphenyl)-C₅-C₆-cycloalkane, Bis-(hydroxyphenyl)-ether, Bis-(hydroxyphenyl)-sulfoxide, Bis-(hydroxyphenyl)-ketone, Bis-(hydroxyphenyl)-sulfone und α,α-Bis-(hydroxyphenyl)-diisopropyl-benzole sowie deren kernbromierte und/oder kernchlorierte Derivate.

Besonders bevorzugte aromatische Verbindungen mit mindestens zwei Hydroxylgruppen sind 4,4'-Dihydroxydiphenyl, Bisphenol-A, 2,4-Bis(4-hydroxyphenyl)-2-methylbutan, 1,1-Bis-(4-hydroxyphenyl)-cyclohexan, 1,1-Bis-(4-hydroxyphenyl)-3.3.5-trimethylcyclohexan, 4,4'-Dihydroxydiphenylsulfid, 4,4'-Dihydroxydiphenylsulfon sowie deren di- und tetrabromierten oder chlorierten Derivate wie beispielsweise 2,2-Bis(3-Chlor-4-hydroxyphenyl)-propan, 2,2-Bis-(3,5-dichlor-4-hydroxyphenyl)-propan oder 2,2-Bis-(3,5-dibrom-4-hydroxyphenyl)-propan. Insbesondere bevorzugt ist 2,2-Bis-(4-hydroxyphenyl)-propan (Bisphenol-A).

Es können die aromatischen Verbindungen mit mindestens zwei Hydroxylgruppen einzeln oder als beliebige Mischungen eingesetzt werden. Die aromatischen Verbindungen mit mindestens zwei Hydroxylgruppen sind literaturbekannt oder nach literaturbekannten Verfahren erhältlich.

Für die Herstellung von thermoplastischen, aromatischen Polycarbonaten geeignete Kettenabbrecher sind beispielsweise Phenol, p-Chlorphenol, p-tert.-Butylphenol oder 2,4,6-Tribromphenol, aber auch langkettige Alkylphenole, wie 4-[2-(2,4,4-Trimethylpentyl)]-phenol, 4-(1,3-Tetramethylbutyl)-phenol gemäß DE-A 2 842 005 oder Monoalkylphenol oder Dialkylphenole mit insgesamt 8 bis 20 Kohlenstoffatomen in den Alkylsubstituenten, wie 3,5-di-tert.-Butylphenol, p-iso-Octylphenol, p-tert.-Octylphenol, p-Dodecylphenol und 2-(3,5-Dimethylheptyl)-phenol und 4-(3,5-Dimethylheptyl)-phenol. Die Menge an einzusetzenden Kettenabbrechern beträgt im allgemeinen zwischen 0,5 Mol%, und 10 Mol%, bezogen auf die Molsumme der jeweils eingesetzten aromatischen Verbindungen mit mindestens zwei Hydroxylgruppen..

Einsetzbare thermoplastische, aromatische Polycarbonate haben im Rahmen einer bevorzugten Ausführungsform der Erfindung mittlere Molekulargewichte (Gewichtsmittel M_{w}, gemessen durch GPC (Gelpermeationschromatographie) mit Polycarbonatstandard auf Basis Bisphenol A) von bevorzugt 20000 bis 40000 g/mol, weiter bevorzugt 24000 bis 32000 g/mol, besonders bevorzugt 26000 bis 30000 g/mol.

Die thermoplastischen, aromatischen Polycarbonate können in bekannter Weise verzweigt sein, und zwar vorzugsweise durch den Einbau von 0,05 bis 2,0 Mol%, bezogen auf die Summe der eingesetzten aromatische Verbindung mit mindestens zwei Hydroxylgruppen, an dreifunktionellen oder mehr als dreifunktionellen Verbindungen, beispielsweise solchen mit drei und mehr phenolischen Gruppen.

Bevorzugt werden lineare Polycarbonate, weiter bevorzugt auf Basis von Bisphenol-A, eingesetzt.

Geeignet sind sowohl Homopolycarbonate als auch Copolycarbonate. Zur Herstellung erfindungsgemäß bevorzugt einsetzbarer Copolycarbonate können auch 1 bis 25 Gew.%, vorzugsweise 2,5 bis 25 Gew.%, bezogen auf die Gesamtmenge an einzusetzender aromatischer Verbindung mit mindestens zwei Hydroxylgruppen, Polydiorganosiloxane mit Hydroxyaryloxy-Endgruppen eingesetzt werden. Diese sind bekannt (US 3 419 634) und nach literaturbekannten Verfahren herstellbar. Ebenfalls geeignet sind Polydiorganosiloxanhaltige Copolycarbonate; die Herstellung der Polydiorganosiloxanhaltiger Copolycarbonate ist beispielsweise in der DE-A 3 334 782 beschrieben.

Aromatische Dicarbonsäuredihalogenide zur Herstellung von aromatischen Polyestercarbonaten sind vorzugsweise die Disäuredichloride der Isophthalsäure, Terephthalsäure, Diphenylether-4,4'-dicarbonsäure und der Naphthalin-2,6-dicarbonsäure.

Besonders bevorzugt sind Gemische der Disäuredichloride der Isophthalsäure und der Terephthalsäure im Verhältnis zwischen 1:20 und 20:1.

Bei der Herstellung von Polyestercarbonaten wird zusätzlich ein Kohlensäurehalogenid, vorzugsweise Phosgen, als bifunktionelles Säurederivat mit verwendet.

Als Kettenabbrecher für die Herstellung der aromatischen Polyestercarbonate kommen außer den bereits genannten Monophenolen noch deren Chlorkohlensäureester sowie die Säurechloride von aromatischen Monocarbonsäuren, die gegebenenfalls durch C₁ bis C₂₂-Alkylgruppen oder durch Halogenatome substituiert sein können, sowie aliphatische C₂ bis C₂₂-Monocarbonsäurechloride in Betracht.
Die Menge an Kettenabbrechern beträgt jeweils 0,1 bis 10 Mol%, bezogen im Falle der phenolischen Kettenabbrecher auf Mol aromatishe Verbindung mit mindestens zwei Hydroxylgruppen und im Falle von Monocarbonsäurechlorid-Kettenabbrecher auf Mol Dicarbonsäuredichlorid.
Bei der Herstellung von aromatischen Polyestercarbonaten kann zusätzlich eine oder mehrere aromatische Hydroxycarbonsäure eingesetzt werden.

Die aromatischen Polyestercarbonate können sowohl linear als auch in bekannter Weise verzweigt sein (siehe dazu DE-A 2 940 024 und DE-A 3 007 934), wobei lineare Polyestercabonate bevorzugt sind.

Als Verzweigungsmittel können beispielsweise drei- oder mehrfunktionelle Carbonsäurechloride, wie Trimesinsäuretrichlorid, Cyanursäuretrichlorid, 3,3'-,4,4'-Benzophenontetracarbonsäuretetrachlorid, 1,4,5,8-Napthalintetracarbon-säuretetrachlorid oder Pyromellithsäuretetrachlorid, in Mengen von 0,01 bis 1,0 Mol% (bezogen auf eingesetzte Dicarbonsäuredichloride) oder drei- oder mehrfunktionelle Phenole, wie Phloroglucin, 4,6-Dimethyl-2,4,6-tri-(4-hydroxyphenyl)-hept-2-en, 4,6-Dimethyl-2,4-6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis[4,4-bis(4-hydroxy-phenyl)-cyclohexyl]-propan, 2,4-Bis(4-hydroxyphenyl-isopropyl)-phenol, Tetra-(4-hydroxyphenyl)-methan, 2,6-Bis(2-hydroxy-5-methyl-benzyl)-4-methyl-phenol, 2-(4-Hydroxyphenyl)-2-(2,4-dihydroxyphenyl)-propan, Tetra-(4-[4-hydroxyphenyl-isopropyl]-phenoxy)-methan, 1,4-Bis[4,4'-dihydroxytriphenyl)-methyl]-benzol, in Mengen von 0,01 bis 1,0 Mol% bezogen auf eingesetzte Diphenole verwendet werden. Phenolische Verzweigungsmittel können mit den Diphenolen vorgelegt werden; Säurechlorid-Verzweigungsmittel können zusammen mit den Säuredichloriden eingetragen werden.

In den thermoplastischen, aromatischen Polyestercarbonaten kann der Anteil an Carbonatstruktureinheiten beliebig variieren. Vorzugsweise beträgt der Anteil an Carbonatgruppen bis zu 100 Mol%, insbesondere bis zu 80 Mol%, besonders bevorzugt bis zu 50 Mol%, bezogen auf die Summe an Estergruppen und Carbonatgruppen. Sowohl der Esterals auch der Carbonatanteil der aromatischen Polyestercarbonate kann in Form von Blöcken oder statistisch verteilt im Polykondensat vorliegen.

In Frage kommende Polyester für die Synthese Polycarbonat haltiger Verbindungen sind in bevorzugter Ausführungsform aromatisch, weiter bevorzugt handelt es sich um Polyalkylenterephthalate. Es handelt sich hierbei in besonders bevorzugter Ausführungsform um Reaktionsprodukte aus aromatischen Dicarbonsäuren oder ihren reaktionsfähigen Derivate, wie Dimethylester oder Anhydride, und aliphatischen, cycloaliphatischen oder araliphatischen Diolen sowie Mischungen dieser Reaktionsprodukte. Besonders bevorzugte aromatische Polyalkylenterephthalate enthalten mindestens 80 Gew.-%, vorzugsweise mindestens 90 Gew.-%, bezogen auf die Dicarbonsäurekomponente Terephthalsäurereste und mindestens 80 Gew.-%, vorzugsweise mindestens 90 Gew-%, bezogen auf die Diolkomponente Ethylenglykol- und/oder Butandiol-1,4-Reste.

Die bevorzugten aromatischen Polyalkylenterephthalate können neben Terephthalsäureresten bis zu 20 mol-%, vorzugsweise bis zu 10 mol-%, Reste anderer aromatischer oder cycloaliphatischer Dicarbonsäuren mit 8 bis 14 C-Atomen oder aliphatischer Dicarbonsäuren mit 4 bis 12 C Atomen enthalten, wie z.B. Reste von Phthalsäure, Isophthalsäure, Naphthalin-2,6-dicarbonsäure, 4,4'-Diphenyldi-carbonsäure, Bernsteinsäure, Adipinsäure, Sebacinsäure, Azelainsäure, Cyclohexandiessigsäure.

Die bevorzugten aromatischen Polyalkylenterephthalate können neben Ethylenglykol bzw. Butan¬diol-1,4-Resten bis zu 20 mol-%, vorzugsweise bis zu 10 mol-%, andere aliphatische Diole mit 3 bis 12 C Atomen oder cycloaliphatische Diole mit 6 bis 21 C Atomen enthalten, z.B. Reste von Propandiol-1,3, 2-Ethylpropandiol-1,3, Neopentylglykol, Pentandiol-1,5, Hexandiol-1,6, Cyclo¬hexan-dimethanol-1,4, 3-Ethyl¬pentandiol-2,4, 2-Methylpentandiol-2,4, 2,2,4-Trimethylpentandiol-1,3, 2-Ethyl¬hexandiol-1,3, 2,2-Diethylpropandiol-1,3, Hexandiol-2,5, 1,4-Di-(β-hydroxyethoxy)-benzol, 2,2-Bis-(4-hydroxycyclohexyl)-propan, 2,4-Dihydroxy-1,1,3,3-tetramethyl-cyclobutan, 2,2-Bis-(4-ß-hydroxyethoxy-phenyl)-propan und 2,2-Bis-(4-hydroxypropoxyphenyl)-propan (DE-A 2 407 674, 2 407 776, 2 715 932).

Die aromatischen Polyalkylenterephthalate können durch Einbau relativ kleiner Mengen 3- oder 4-wertiger Alkohole oder 3- oder 4-basischer Carbonsäuren, z.B. gemäß DE-A 1 900 270 und US-PS 3 692 744, verzweigt werden. Beispiele bevorzugter Verzweigungsmittel sind Trimesinsäure, Trimellithsäure, Trimethylolethan und -propan und Pentaerythrit.

Besonders bevorzugt sind aromatische Polyalkylenterephthalate, die allein aus Terephthalsäure und deren reaktionsfähigen Derivaten (z.B. deren Dialkylestern) und Ethylenglykol und/oder Butandiol-1,4 hergestellt worden sind, und Mischungen dieser Polyalkylenterephthalate.

Bevorzugte Mischungen von aromatischen Polyalkylenterephthalaten enthalten 1 bis 50 Gew.-%, vorzugsweise 1 bis 30 Gew.-%, Polyethylenterephthalat und 50 bis 99 Gew.-%, vorzugsweise 70 bis 99 Gew.-%, Polybutylenterephthalat. Die vorzugsweise verwendeten aromatischen Polyalkylenterephthalate besitzen eine Viskositätszahl von 0,4 bis 1,5 dl/g, vorzugsweise 0,5 bis 1,2 dl/g, gemessen in Phenol/o-Dichlorbenzol (1:1 Gewichtsteile) in einer Konzentration von 0,05g/ml gemäß ISO 307 bei 25°C im Ubbelohde-Viskosimeter.

Die aromatischen Polyalkylenterephthalate lassen sich nach bekannten Methoden herstellen (s. z.B. Kunststoff-Handbuch, Band VIII, S. 695 ff., Carl-Hanser-Verlag, München 1973).

Die aromatischen Polycarbonate und Polyestercarbonate können in dem besagten Material des erfindungsgemäßen Verfahrens allein oder im beliebigen Gemisch eingesetzt werden.

Das besagte Material des Pyrolysgutes enthält neben mindestens einer Polycarbonat enthaltenden Verbindung zusätzlich mindestens eine Polystyrol enthaltende Verbindung. Unter "Polystyrol enthaltende Verbindung" wird erfindungsgemäß eine Verbindung verstanden, die eine Wiederholungseinheit abgeleitet von gegebenenfalls am aromatischen Ring substituiertem Styrol (wie insbesondere Styrol, α-Methylstyrol, p-Methylstyrol, p-Chlorstyrol) enthalten.

In einer Ausführungsform des erfindungsgemäßen Verfahrens wird vorteilhafterweise im Pyrolysegut solches Material eingesetzt, das zusätzlich zu mindestens einer Polycarbonat enthaltenden Verbindung mindestens ein Polymer als Polystyrol enthaltende Verbindung enthält, das mindestens zehn Wiederholungseinheiten der Formel

*-CR¹Ph-CH₂-*

enthält, worin * eine Valenz der Wiederholungseinheit des Polymerrückgrats kennzeichnet, R¹ für ein Wasserstoffatom oder eine Methylgruppe und Ph eine, gegebenenfalls mit mindestens einer Gruppe ausgewählt aus (C₁ bis C₄)-Alkylgruppe, Halogenatom (insbesondere Chlor) substituierte, Phenylgruppe bedeutet. Besonders bevorzugt enthält besagtes Material zusätzlich zu mindestens einer Polycarbonat enthaltenden Verbindung mindestens ein Polymer als Polystyrol enthaltende Verbindung, das mindestens zehn Wiederholungseinheiten obiger Formel, abgeleitet von Styrol (R¹ = H, Ph = Phenyl), α-Methylstyrol (R¹ = Methyl, Ph = Phenyl), p-Methylstyrol (R¹ = H, Ph = 4-Methyl-phenyl), p-Chlorstyrol (R¹ = H, Ph = 4-Chlorphenyl) oder von Mischungen daraus.

Als erfindungsgemäß bevorzugt einsetzbares Material, enthält es neben mindestens einer Polycarbonat enthaltenden Verbindung zusätzlich als Polystyrol enthaltende Verbindung mindestens ein Polymer, ausgewählt aus kautschukmodifizierten Pfropfpolymerisaten (B1) mit Polystyrol oder kautschukfreien Vinyl(Co)Polymerisaten mit Polystyrol (B2) oder um eine Mischung mehrerer solcher Polymerisate.

Solche bevorzugt zum Einsatz kommenden kautschukmodifizierten Pfropfpolymerisate (B1) umfassen
B.1.15 bis 95, vorzugsweise 15 bis 92, insbesondere 25 bis 60 Gew.-%, bezogen auf Komponente B.1, wenigstens eines Vinylaromaten und/oder kernsubstituierten Vinylaromaten (wie Styrol, α-Methylstyrol, p-Methylstyrol, p-Chlorstyrol) auf
B.1.2 95 bis 5, vorzugsweise 85 bis 8, insbesondere 75 bis 40 Gew.-%, bezogen auf Komponente B.1, einer oder mehrerer kautschukartiger Pfropfgrundlagen, vorzugsweise mit Glasübergangstemperaturen < 10°C, weiter bevorzugt < 0°C, besonders bevorzugt < -20°C.

Die Glasübergangstemperatur wird mittels dynamischer Differenzkalorimetrie (DSC) gemäß der Norm DIN EN 61006 bei einer Heizrate von 10 K/min mit Definition der T_{g} als Mittelpunkttemperatur (Tangentenmethode) bestimmt.

Die Pfropfgrundlage B. 1.2 hat im Allgemeinen eine mittlere Teilchengröße (dso-Wert) von 0,05 bis 10 µm, vorzugsweise 0,1 bis 5 µm, besonders bevorzugt 0,2 bis 1 µm. Die mittlere Teilchengröße d₅₀ ist der Durchmesser, oberhalb und unterhalb dessen jeweils 50 Gew.-% der Teilchen liegen. Er kann mittels Ultrazentrifugenmessung (W. Scholtan, H. Lange, Kolloid, Z. und Z. Polymere 250 (1972), 782-1796) bestimmt werden.

Monomere B.1.1 sind vorzugsweise Gemische aus
B.1.1.1 50 bis 99, bevorzugt 60 bis 80, insbesondere 70 bis 80 Gew.-Teile, bezogen auf B.1.1, Vinylaromaten und/oder kernsubstituierten Vinylaromaten (insbesondere ausgewählt aus Styrol, α-Methylstyrol, p-Methylstyrol, p-Chlorstyrol oder Mischungen daraus), und
B.1.1.2 1 bis 50, bevorzugt 20 bis 40, insbesondere 20 bis 30 Gew.-Teilen, bezogen auf B.1.1, Vinylcyanide (ungesättigte Nitrile wie Acrylnitril und Methacrylnitril) und/oder (Meth)Acrylsäure-(C₁-C₈)-Alkylester, wie Methylmethacrylat, n-Butylacrylat, t-Butylacrylat, und/oder Derivate (wie Anhydride und Imide) ungesättigter Carbonsäuren, beispielsweise Maleinsäureanhydrid und N-Phenyl-Maleinimid.

Bevorzugte Monomere B.1.1.1 sind ausgewählt aus mindestens einem der Monomere Styrol und α-Methylstyrol, bevorzugte Monomere B.1.1.2 sind ausgewählt aus mindestens einem der Monomere Acrylnitril, Maleinsäureanhydrid und Methylmethacrylat. Besonders bevorzugte Monomere sind B.1.1.1 Styrol und B.1.1.2 Acrylnitril.

Für die Pfropfpolymerisate B.1 geeignete Pfropfgrundlagen B.1.2 sind beispielsweise Dienkautschuke, EP(D)M-Kautschuke, also solche auf Basis Ethylen/Propylen und gegebenenfalls Dien, Acrylat-, Polyurethan-, Silikon-, Chloropren- und Ethylen/Vinylacetat-Kautschuke sowie Silikon/Acrylat-Kompositkautschuke.

Bevorzugte Pfropfgrundlagen B.1.2 sind Dienkautschuke, beispielsweise auf Basis Butadien und Isopren, oder Gemische von Dienkautschuken oder Copolymerisate von Dienkautschuken oder deren Gemischen mit weiteren copolymerisierbaren Monomeren (z.B. gemäß B.1.1.1 und B.1.1.2).

Besonders bevorzugt als Pfropfgrundlage B.1.2 ist reiner Polybutadienkautschuk.

Besonders bevorzugte Polymerisate B.1 sind beispielsweise ausgewählt aus Copolymerisat aus Acrylnitril-Butadien-Styrol (auch als ABS-Polymerisat bezeichnet) oder Copolymerisat aus Methacrylat-Butadien-Styrol (auch als MBS-Polymerisat bezeichnet), wie sie z.B. in der DE-OS 2 035 390 (=US-PS 3 644 574) oder in der DE-OS 2 248 242 (=GB-PS 1 409 275) bzw. in Ullmanns, Enzyklopädie der Technischen Chemie, Bd. 19 (1980), S. 280 ff. beschrieben sind.

Die Pfropfcopolymerisate B.1 werden durch radikalische Polymerisation, z.B. durch Emulsions-, Suspensions-, Lösungs- oder Massepolymerisation, vorzugsweise durch Emulsions- oder Massepolymerisation, insbesondere durch Emulsionspolymerisation hergestellt.

Der Gelanteil der Pfropfgrundlage B.1.2 beträgt mindestens 30 Gew.-%, vorzugsweise mindestens 40 Gew.-%, insbesondere mindestens 60 Gew.-%, jeweils bezogen auf B.1.2 und gemessen als unlöslicher Anteil in Toluol.

Der Gelgehalt der Pfropfgrundlage B.1.2 wird bei 25°C in einem geeigneten Lösungsmittel als in diesen Lösungsmitteln unlöslicher Anteil bestimmt (M. Hoffmann, H. Krömer, R. Kuhn, Polymeranalytik I und II, Georg Thieme-Verlag, Stuttgart 1977).

Besonders geeignete Pfropfkautschuke sind auch ABS-Polymerisate, die durch Redox-Initiierung mit einem Initiatorsystem aus organischem Hydroperoxid und Ascorbinsäure gemäß US-P 4 937 285 hergestellt werden.

Da bei der Pfropfreaktion die Pfropfmonomeren bekanntlich nicht unbedingt vollständig auf die Pfropfgrundlage aufgepfropft werden, werden erfindungsgemäß unter Pfropfpolymerisaten B.1 auch solche Produkte verstanden, die durch (Co)Polymerisation der Pfropfmonomere in Gegenwart der Pfropfgrundlage gewonnen werden und bei der Aufarbeitung mit anfallen. Diese Produkte können demnach auch freies, d.h. nicht chemisch an den Kautschuk gebundenes (Co)Polymerisat der Pfropfmonomere enthalten.

Zur Vernetzung können Monomere mit mehr als einer polymerisierbaren Doppelbindung copolymerisiert werden. Bevorzugte Beispiele für vernetzende Monomere sind Ester ungesättigter Monocarbonsäuren mit 3 bis 8 C-Atomen und ungesättigter einwertiger Alkohole mit 3 bis 12 C-Atomen, oder gesättigter Polyole mit 2 bis 4 OH-Gruppen und 2 bis 20 C-Atomen, wie Ethylenglykoldimethacrylat, Allylmethacrylat; mehrfach ungesättigte heterocyclische Verbindungen, wie Trivinyl- und Triallylcyanurat; polyfunktionelle Vinylverbindungen, wie Di- und Trivinylbenzole; aber auch Triallylphosphat und Diallylphthalat. Bevorzugte vernetzende Monomere sind Allylmethacrylat, Ethylenglykoldimethacrylat, Diallylphthalat und heterocyclische Verbindungen, die mindestens drei ethylenisch ungesättigte Gruppen aufweisen. Besonders bevorzugte vernetzende Monomere sind die cyclischen Monomere Triallylcyanurat, Triallylisocyanurat, Triacryloylhexahydro-s-triazin, Triallylbenzole. Die Menge der vernetzten Monomere beträgt vorzugsweise 0,02 bis 5, insbesondere 0,05 bis 2 Gew.-%, bezogen auf die Pfropfgrundlage B.1.2. Bei cyclischen vernetzenden Monomeren mit mindestens drei ethylenisch ungesättigten Gruppen ist es vorteilhaft, die Menge auf unter 1 Gew.-% der Pfropfgrundlage B.1.2 zu beschränken.

Bevorzugte "andere" polymerisierbare, ethylenisch ungesättigte Monomere, die neben den Acrylsäureestern gegebenenfalls zur Herstellung der Pfropfgrundlage B.1.2 dienen können, sind z.B. Acrylnitril, Styrol, α-Methylstyrol, Acrylamide, Vinyl-C₁-C₆-alkylether, Methylmethacrylat, Butadien. Bevorzugte Acrylatkautschuke als Pfropfgrundlage B.1.2 sind Emulsionspolymerisate, die einen Gelgehalt von mindestens 60 Gew.-% aufweisen.

Weitere geeignete Pfropfgrundlagen gemäß B.1.2 sind Silikonkautschuke mit pfropfaktiven Stellen, wie sie in den DE-OS 3 704 657, DE-OS 3 704 655, DE-OS 3 631 540 und DE-OS 3 631 539 beschrieben werden.

Bei den kautschukfreien Vinyl(Co)Polymerisaten gemäß Komponente B.2 handelt es sich vorzugsweise um kautschukfreie Homo- und/oder Copolymerisate von mindestens einem Monomeren aus der Gruppe der Vinylaromaten, Vinylcyanide (ungesättigte Nitrile), (Meth)-Acrylsäure-(C₁ bis C₈)-Alkylester, ungesättigten Carbonsäuren sowie Derivaten (wie Anhydride und Imide) ungesättigter Carbonsäuren.

Insbesondere geeignet sind (Co)Polymerisate B.2 aus
B.2.1 50 bis 99 Gew.-%, bevorzugt 60 bis 80 Gew.-%, insbesondere 70 bis 80 Gew.-%, jeweils bezogen auf das Gesamtgewicht des (Co)Polymerisats B.2, mindestens eines Monomeren ausgewählt aus der Gruppe der Vinylaromaten, wie beispielsweise Styrol, α-Methylstyrol, kernsubstituierten Vinylaromaten, wie beispielsweise p-Methylstyrol, p-Chlorstyrol, (und gegebenenfalls zusätzlich (Meth)Acrylsäure-(C₁-C₈)-Alkylester, wie beispielsweise Methylmethacrylat, n-Butylacrylat, tert.-Butylacrylat), und
B.2.2 1 bis 50 Gew.-%, bevorzugt 20 bis 40 Gew.-%, insbesondere 20 bis 30 Gew.-%, jeweils bezogen auf das Gesamtgewicht des (Co)Polymerisats B.2, mindestens eines Monomeren ausgewählt aus der Gruppe der Vinylcyanide, wie beispielsweise ungesättigte Nitrile wie z.B. Acrylnitril und Methacrylnitril, (Meth)Acrylsäure-(C₁-C₈)-Alkylester, wie beispielsweise Methylmethacrylat, n-Butylacrylat, tert.-Butylacrylat, ungesättigte Carbonsäuren und Derivate ungesättigter Carbonsäuren, wie beispielsweise Maleinsäureanhydrid und N-Phenyl-Maleinimid.

Diese (Co)Polymerisate B.2 sind harzartig, thermoplastisch und kautschukfrei. Besonders bevorzugt ist zumindest das Copolymerisat aus B.2.1 Styrol und B.2.2 Acrylnitril in besagtem Material enthalten.

Derartige (Co)Polymerisate B.2 sind bekannt und lassen sich durch radikalische Polymerisation, insbesondere durch Emulsions-, Suspensions-, Lösungs- oder Massepolymerisation herstellen. Die (Co)Polymerisate besitzen vorzugsweise mittlere Molekulargewichte M_{w} (Gewichtsmittel, ermittelt durch GPC mit Polystyrol als Standard) zwischen 15.000 und 250.000 g/mol, bevorzugt im Bereich 80.000 bis 150.000 g/mol.

Besagtes Material des Pyrolysegutes kann neben mindestens einer Polycarbonat enthaltenden Verbindung und mindestens einer Polystyrol enthaltenden Verbindung zusätzlich mindestens ein Polymeradditiv enthalten, ausgewählt aus Flammschutzmitteln, Antidrippingmitteln, Flammschutzsynergisten, Rauchinhibitoren, Gleit- und Entformungsmitteln, Nukleiermitteln, Antistatika, Leitfähigkeitsadditiven, Stabilisatoren (z.B. Hydrolyse-, Wärmealterungs- und UV-Stabilisatoren sowie Umesterungsinhibitoren), Fließfähigkeitspromotoren, Phasenverträglichkeitsvermittlern, weiteren von Komponenten A und B verschiedenen polymeren Bestandteilen (beispielsweise funktionelle Blendpartnern), Füll- und Verstärkungsstoffen sowie Farbstoffen und Pigmenten.

In bevorzugter Ausführungsform enthält besagtes Material mindestens ein Polymeradditiv ausgewählt aus der Gruppe bestehend aus Gleit- und Entformungsmitteln, Stabilisatoren (wie beispielsweise mindestens einen Stabilisator ausgewählt aus sterisch gehindertem Phenol, organischem Phosphit, Schwefel-basiertem Co-Stabilisator oder Mischungen daraus), Fließfähigkeitspromotoren, Phasenverträglichkeitsvermittlern, weiteren polymeren Bestandteilen, Farbstoffen und Pigmenten.

In bevorzugter Ausführungsform enthält die Zusammensetzung als Entformungsmittel Pentaerythrittetrastearat.

Die Dosierung und Prozessierbarkeit des Pyrolysegutes in der Pyrolyse des erfindungsgemäßen Verfahrens lässt sich vereinfachten, indem besagtes Pyrolysegut im Schritt (a) neben besagtem Material zusätzlich mindestens einen Füllstoff enthält. Besagter Füllstoff katalysiert bevorzugt nicht im Rahmen der Pyrolyse die thermische Zersetzung von Polycarbonat. Dabei ist es wiederum besonders bevorzugt, wenn der Füllstoff mindestens ein Metalloxid ist, das im Rahmen der Pyrolyse der thermischen Zersetzung von Polycarbonat nicht katalytisch wirksam ist, welches bevorzugt ausgewählt aus SiO₂.

Das Polycarbonat Material wird vorzugsweise mit einem Füllstoff z.B. Sand gemischt, wodurch eine kontinuierliche Prozessführung des erfindungsgemäßen Verfahrens vereinfacht wird. Insbesondere wird ein Verkleben des besagten Materials im Reaktor und in der Zuführung der Dosiervorrichtung zum Reaktor vermieden. Im Rahmen einer Ausführungsform wird der Füllstoff und das besagte Material im Volumenverhältnis Füllstoff zu besagtem Material von mindestens 0,1 zu 1 bis 10 zu 1 dem Pyrolysegut als Gemisch zugeführt.

Um den Pyrolyse Prozess selektiver und effizienter durchzuführen, können Katalysatoren eingesetzt werden. Eine effektivere und selektivere Zersetzung wird erhalten, wenn in einer Ausführungsform des erfindungsgemäßen Verfahrens das besagte im Schritt (a) eingebrachte Pyrolysegut neben besagtem Material zusätzlich mindestens einen, die Reaktion der Zersetzung des besagten Materials beeinflussenden Katalysator enthält. Ein entsprechender Katalysator kann die Pyrolysetemperatur herabsetzen, das Produktspektrum auf gewünschte Produkte reduzieren und gegebenenfalls die Verkokung minimieren. Für einen effizienten Prozess sind kostengünstige Katalysatoren bevorzugt. Dies können beispielsweise natürlich vorkommende Materialien wie anorganische Salze, feuerfeste Oxide, Mineralien und Industriesteine, in die optional Ionen in einem einfachen Ionenaustauschprozess ausgetauscht werden können, sein, da sie keine umfangreiche Synthese erfordern. Sie sind leicht verfügbar und sind daher relativ billig. Synthetische Katalysatoren wie Zeolithe (ZSM-5, ZeolithX,Y, etc) sind dagegen ein Beispiel für Katalysatoren, die zwar effektiv, aber nicht billig sind, da sie speziell hergestellt werden müssen.

Neben der Funktion als Katalysator können Katalysatoren ebenfalls die Dosierung und Prozessierbarkeit des Pyrolysegutes in der Pyrolyse vereinfachen. Bei Einsatz eines Katalysators kann in diesem Fall die eingesetzte Menge an Füllstoff reduziert werden. Im Rahmen einer Ausführungsform wird die Gesamtmenge an Füllstoff und Katalysator im Verhältnis zur Menge des besagten Materials im Volumenverhältnis von mindestens 0,1 zu 1 bis 10 zu 1 dem Pyrolysegut als Gemisch zugeführt.

Bei der Pyrolysereaktion wird vorzugsweise mindestens ein Katalysator ausgewählt aus der Gruppe, die gebildet wird aus alkalischen anorganischen Materialien, bevorzugter aus der oben definierten Gruppe von natürlich vorkommenden Materialien. Diese anorganischen Materialien können feuerfeste Oxide sein. Feuerfeste Oxide sind Metalloxide, die bei hohen Temperaturen von 300°C bis 700°C stabil sind. Solche als Katalysator fungierenden Oxide umfassen die Oxide von Aluminium, Magnesium, Zirkonium, Titan, Chrom, Zink, Zinn und andere Metalle oder Kombinationen von Aluminiumoxid mit Magnesiumoxid und oder Calciumoxid.

Kristalline anorganische Materialien umfassen Aluminosilikate, SiliciumAluminiumphosphate, Silicalit, Spinelle und andere natürliche Zeolithe und Tone. Als besagter Katalysator eignet sich somit insbesondere mindestens eine Verbindung, aus der Gruppe, die gebildet wird aus anorganischen Salzen, Mineralien, Metalloxiden, Mischoxiden, Tonen, Zeolithen.

Es können sowohl homogene als auch heterogene Katalysatoren eingesetzt werden. Es sind jedoch solche Ausführungsformen des erfindungsgemäßen Verfahrens bevorzugt, in denen der Katalysator ein heterogener Katalysator ist. Es hat sich als vorteilhaft erwiesen, wenn der Katalysator bevorzugt in Form festförmiger Teilchen (insbesondere in Form eines körnigen Gemenges) in den Reaktor eingebracht wird. Der eingesetzte heterogene Katalysator weist besonders bevorzugt eine mittlere Teilchengröße seiner festförmigen Teilchen, insbesondere seiner losen, festförmigen Partikel des körnige Gemenges, einen mittleren Durchmesser X_{50,3} (Volumenmittel) von 0,01 mm bis 5 cm, bevorzugt von 0,1 mm bis 5 cm, auf. Der mittlere Teilchengrößendurchmesser X_{50,3} wird durch Siebung oder mittels eines Partikelgrößenanalysators Camsizer der Fa. Retsch bestimmt.

Bei Einsatz eines heterogenen Katalysators hat es sich im Rahmen einer weiteren bevorzugten Ausführungsform als besonders geeignet erwiesen, wenn die Katalysatorpartikel eine kleinere oder die gleiche Partikelgröße besitzen, wie die Partikel des besagten Materials. Daher ist es bevorzugt, wenn im Pyrolysegut die mittlere Teilchengröße des darin befindlichen Katalysators höchstens der mittleren Teilchengröße des darin befindlichen besagten Materials entspricht.

Als besonders bevorzugt geeigneter, besagter Katalysator kann das Pyrolysegut mindestens einen basischen Katalysator enthalten. Handelt es sich dabei um einen festförmigen, basischen Katalysator, kann die Anzahl und Stärke der basischen Zentren des Katalysators mittels Fourier transform Infrarotspektroskopie und Temperature Programmed Desorption von CO₂ (TPD-CO2), oder mittels gängiger titrimetrischer Methoden bestimmt werden.

Wird ein Katalysator zugesetzt, kann dieser dem Füllmaterial beigemischt, auf das Füllmaterial aufgezogen, oder als Ersatz des Füllmaterials eingesetzt werden.

Da im erfindungsgemäßen Verfahren eingesetzter Katalysator dazu neigt, Ablagerungen von Koks und anderen Kohlenstoffrückständen zu erhalten, wird bei Einsatz von Katalysator bevorzugt ein Reaktor eingesetzt, der einfach und vorzugsweise die Ausbringung und kontinuierliche Regeneration des Katalysators ermöglicht. Daher ist die Verwendung eines kontinuierlichen Rührkesselreaktors (CSTR), eines bewegliches Bettes, eines Schneckenreaktors, eines Schneckenfördererreaktors, eines Flugstromreaktors, eines Drehkegelreaktors oder eines fluidisierten Bettreaktors gegenüber der Verwendung eines Festbettreaktors bevorzugt.

Der (z.B. durch Verkokung) deaktivierte Katalysator befindet sich im Pyrolyserückstand und kann nach der Ausbringung des Pyrolyserückstandes aus dem Reaktor in einen Regenerator geleitet und nach der Regeneration dem in den Pyrolysereaktor einzubringenden Pyrolysegut des Schritts (b) zugeschlagen werden. Reaktoren, die eine kurze Kontaktzeit, ein intensives Mischen der Komponenten im Beschickungsstrom mit dem Katalysator und für eine kontinuierliche Rückführung des regenerierten Katalysators in die Pyrolysezone erlauben, sind am meisten bevorzugt. Da dies bei einem Schneckenreaktor, Drehrohrofen oder Wirbelbett der Fall ist, sind diese Reaktoren besonders bevorzugt.

Das in Schritt (a) in den Reaktor eingebrachte Pyrolysegut wird gemäß Schritt (b) unter Bildung von Pyrolysat und Pyrolyserückstand zumindest teilweise zersetzt.
Das Pyrolysegut wird nach dessen Einbringung in den Reaktor auf eine Temperatur im Bereich von 300°C bis 700°C aufgeheizt. Eine besonders erfolgreiche Verbesserung des Ergebnisses des erfindungsgemäßen Verfahrens lässt sich erreichen, wenn im Rahmen einer Ausführungsform das eingebrachte Pyrolysegut auf 300°C bis 700°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes 1 Sekunde bis 2 Stunden, bevorzugt zwischen 2 Minuten und 60 Minuten, beträgt, und wobei die Temperatur und der Gehalt von Sauerstoffgas im Reaktor während dieses Zeitraums die in Schritt (b) definierten Werte betragen.

Enthält in dem erfindungsgemäßen Verfahren das Pyrolysegut einen Katalysator, befindet sich dieser Katalysator im ausgebrachten Pyrolyserückstand. Im Rahmen einer weiteren Ausführungsform des Verfahrens, wird daher der ausgebrachte Pyrolyserückstand wie oben beschrieben einem Regenerationsschritt des darin enthaltenen Katalysators zugeführt.

Davon unabhängig lassen sich gute Ergebnisse erzielen, wenn die in Schritt (b) stattfindende Herausführung des Pyrolysates aus dem Reaktor durch einen durch den Reaktor geführten Gasstrom oder durch Sog gewährleistet wird und weiter bevorzugt dadurch eine Verweildauer des Pyrolysates als Zeitraum zwischen dem Zeitpunkt der Einbringung des besagten im Schritt (a) in den Reaktor eingebrachten Materials und dem Zeitpunkt der Ausbringung des Pyrolysates 0,1 Sekunden bis 10 Sekunden, bevorzugt zwischen 0,5 Sekunden und 5 Sekunden, besonders bevorzugt 0,5 Sekunden bis 2 Sekunden, beträgt.
Wird ein Gasstrom zu diesem Zweck durch den Reaktor geführt, eignet sich als Gas für diesen Gasstrom insbesondere ein Inertgas, bevorzugt ausgewählt aus Stickstoff, Argon, CO₂, NO oder einem Gemisch daraus.

Wird ein Gasstrom zur Ausbringung des Pyrolysates genutzt, ist es erfindungsgemäß bevorzugt, wenn die Fließgeschwindigkeit des Gasstromes im Reaktor als Leerrohrgeschwindigkeit im Bereich von 0,01 m/s bis 20 m/s liegt. Sollte als Reaktor ein Festbettreaktor gewählt werden, ist es erfindungsgemäß bevorzugt, wenn die Fließgeschwindigkeit des Gasstromes im Reaktor als Leerrohrgeschwindigkeit im Bereich von 0,03 m/s bis 1 m/s liegt. Sollte als Reaktor ein Wirbelbettreaktor gewählt werden, ist es erfindungsgemäß bevorzugt, die Fließgeschwindigkeit des Gasstromes im Reaktor als Leerrohrgeschwindigkeit im Bereich von 0,5 m/s bis 2 m/s liegt. Sollte als Reaktor ein Flugstromreaktor gewählt werden, ist es erfindungsgemäß bevorzugt, die Fließgeschwindigkeit des Gasstromes im Reaktor als Leerrohrgeschwindigkeit im Bereich von 5 m/s bis 20 m/s liegt.

Als erfindungsgemäße Bedingungen für die Zersetzung im Rahmen der Pyrolyse in Schritt (b) beträgt die Temperatur im Reaktor 300 bis 700 °C und die Menge an Sauerstoffgas im Reaktor beträgt von 0 bis 2,0 Vol.% bezogen auf das Gesamtvolumen der im Reaktor befindlichen Gase.

Für die Einstellung der erfindungsgemäßen Menge an Sauerstoffgas, wird der mit besagtem Material des besagten Pyrolysegutes befüllte Reaktor mit Inertgas, insbesondere mit Stickstoff, Argon, CO₂, NO oder einer Mischung daraus befüllt. Dem Inertgas können zusätzlich von Sauerstoffgas verschiedene reaktive Gase beigemischt werden, insbesondere ausgewählt aus Methan, gasförmigem H₂O, Wasserstoffgas oder Gemischen daraus.

Um durch die Einbringung des Pyrolysegutes in den Reaktor das Eintragen von Sauerstoffgas zu minimieren, kann das Pyrolysegut vor dessen Einbringung in Schritt (a) von Sauerstoffgas befreit werden, z.B. durch Austreiben des Sauerstoffgases mittels einer Strippung mit einem Strippgas, z.B. in einem dem Reaktor vorgeschalteten Vorlagebehälter. Beispielsweise könnte als Strippgas Inertgas, insbesondere Stickstoff, Argon, CO₂, NO, Mischungen daraus, von oben oder von unten im Vorlagebehälter (bevorzugt von oben) über eine Fritte in den Behälter dem Pyrolysegut zu geführt werden, um so das Sauerstoffgas auszutreiben.

Im Rahmen einer bevorzugten Ausführungsform des erfindungsgemäßen Verfahrens beträgt die Temperatur im Schritt (b) von 350°C bis 650°C, bevorzugt zwischen 400°C und 650°C, besonders bevorzugt von 420°C bis 600°C, ganz besonders bevorzugt von 450°C bis 580°C.

Im Rahmen einer weiteren bevorzugten Ausführungsform des erfindungsgemäßen Verfahrens beträgt im Schritt (b) die Menge an Sauerstoffgas im Reaktor höchstens 0,5 Vol.%, bevorzugt 0,1 Vol.%, jeweils bezogen auf das Gesamtvolumen der im Reaktor befindlichen Gase.

Im Rahmen einer ganz bevorzugten Ausführungsform des erfindungsgemäßen Verfahrens beträgt im Schritt (b) erstens die Temperatur von 350°C bis 650°C, bevorzugt zwischen 400°C und 650°C, besonders bevorzugt von 420°C bis 600°C, ganz besonders bevorzugt von 450°C bis 580°C, und beträgt zweitens die Menge an Sauerstoffgas im Reaktor höchstens 0,5 Vol.%, bevorzugt 0,1 Vol.%, jeweils bezogen auf das Gesamtvolumen der im Reaktor befindlichen Gase.

Eine bevorzugte Ausführungsform des Verfahrens liefert eine kontinuierliche Prozessführung. Dafür laufen zumindest die Schritte (a) und (b) im Rahmen einer kontinuierlichen Prozessführung gleichzeitig ab.

Das gemäß Schritt (c) erhaltene Pyrolyseprodukt kann mit gängigen Trennungsmethoden aufgearbeitet werden und dadurch (i) mindestens eine aromatische Verbindung mit mindestens zwei Hydroxylsubstituenten, bevorzugt von Bisphenol-A, und (ii) mindestens eine aromatischen Verbindung mit mindestens einem Vinylsubstituenten, bevorzugt von Styrol, erhalten werden. Standardmäßig wird das BPA durch Kristallisation des BPA/Phenol Adduktes gewonnen oder durch Fällen in Toluol erhalten werden Andere Möglichkeiten bestehen in der Destillation oder Extraktion des BPA aus dem Pyrolyseprodukt Gemisch. Andere Produkte wie Styrol oder Phenol können durch Destillation getrennt werden.

Das erfindungsgemäße Verfahren kann mit Hilfe einer entsprechend ausgestalteten Pyrolysevorrichtung durchgeführt werden. Ein weiterer Gegenstand der Erfindung ist somit eine Pyrolysevorrichtung für die Erzeugung von Pyrolysat aus Pyrolysegut, enthaltend mindestens eine Dosiervorrichtung zur Einspeisung von Pyrolysegut, mindestens einen heizbaren Reaktor für die Pyrolyse, und mindestens einen Pyrolysat-Kollektor, dadurch gekennzeichnet, dass
besagter heizbarer Reaktor für die Pyrolyse mindestens eine Heizung enthält, die zur Temperierung des Reaktors auf eine Temperatur von 300°C bis 700°C verwendet werden kann, und mindestens einen Einlass für Pyrolysegut enthält und mindestens einen davon verschiedenen Auslass für Pyrolysat enthält; und

Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
mindestens ein Pyrolysat-Kollektor mindestens eine auf eine Temperatur unter 300°C temperierte Kühlvorrichtung enthält, die im besagten Kollektor zur Senkung der Temperatur des aus dem besagten Reaktor herausgeführten Pyrolysates auf weniger als 300°C unter Bildung von Pyrolyseprodukt, ausgewählt aus Pyrolysatkondensat, Pyrolysatresublimat oder einer Mischung daraus, verwendbar ist, und mindestens einen Behälter zur Sammlung und Ausbringung des unter Kühlung erhaltenen Pyrolyseprodukts enthält; und
mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können.

Als Heizung des heizbaren Reaktors kein beispielsweise ein Heizelement, z.B. Heizwendel oder Heizplatten, dienen oder eine Vorrichtung zur Beheizung eines Gasstromes und zur Einbringung des beheizten Gasstroms in den Reaktor.

Der Reaktor enthält im Rahmen einer bevorzugten Ausführungsform zusätzlich mindestens einen Anschluss zu einer Gasquelle, wobei über eine Regelung, z.B. ein Ventil, ein Gasstrom im Reaktor, bevorzugt mit einer Fließgeschwindigkeit als Leerrohrgeschwindigkeit von zwischen 0,01 m/s bis20 m/s, durch den Reaktor in den Pyrolysat-Kollektor fließt. Sollte als Reaktor ein Festbettreaktor gewählt werden, ist es erfindungsgemäß bevorzugt, wenn die Fließgeschwindigkeit des Gasstromes im Reaktor als Leerrohrgeschwindigkeit im Bereich von 0,03 m/s bis 1 m/s liegt. Sollte als Reaktor ein Wirbelbettreaktor gewählt werden, ist es erfindungsgemäß bevorzugt, die Fließgeschwindigkeit des Gasstromes im Reaktor als Leerrohrgeschwindigkeit im Bereich von 0,5 m/s bis 2 m/s liegt. Sollte als Reaktor ein Flugstromreaktor gewählt werden, ist es erfindungsgemäß bevorzugt, die Fließgeschwindigkeit des Gasstromes im Reaktor als Leerrohrgeschwindigkeit im Bereich von 5 m/s bis 20 m/s liegt. Der Gasstrom aus der Gasquelle kann beispielsweise vor der Einbringung in den Reaktor wie zuvor beschrieben durch die Heizung beheizt werden.

Der Pyrolysat-Kollektor der erfindungsgemäßen Vorrichtung enthält bevorzugt eine Kühlvorrichtung, die im besagten Kollektor zur Senkung der Temperatur des aus dem besagten Reaktor herausgeführten Pyrolysates auf weniger als 50°C (weiter bevorzugt auf weniger als 30°C) unter Bildung von Pyrolyseprodukt verwendbar ist. Hierfür eignen sich bevorzugt Kühlaggregate die nach dem Wärmetauscherprinzip arbeiten.

Unter einer "Fluidverbindung" wird erfindungsgemäß ein Teil der Vorrichtung verstanden, der Anlagenteile miteinander verbindet und durch die sich ein Stoff, der in jedem Aggregatzustand vorliegen kann, von einem Anlagenteil zum nächsten transportieren lässt, beispielsweise eine Zuleitung in Form eines Rohres.

Ein weiterer Gegenstand der Erfindung ist die Verwendung einer Pyrolyse-Vorrichtung des vorgenannten Erfindungsgegenstandes zur Rückgewinnung von bei der Herstellung von Polycarbonat enthaltenden Verbindungen eingesetzter, organischer Verbindung mit mindestens zwei Hydroxylgruppen bei gleichzeitiger Rückgewinnung von bei der Herstellung von Polystyrol-haltiger Verbindung verwendetem Styrol, durch simultaner Pyrolyse besagter Polycarbonat enthaltender Verbindung und besagter Polystyrol-haltigen Verbindung.

Im Rahmen einer bevorzugten Ausführungsform der Verwendung wird die Pyrolyse-Vorrichtung neben der besagten gleichzeitigen Styrol-Rückgewinnung zur Rückgewinnung von bei der Herstellung von Polycarbonat enthaltender Verbindung eingesetztem Bisphenol-A verwendet.

Das erfindungsgemäße Verfahren, sowie die erfindungsgemäße Vorrichtung tragen zur Lösung der zuvor gestellten Aufgabe bei und liefern nach Schritt (c) des Verfahrens als Pyrolyseprodukt eine Zusammensetzung, mindestens enthaltend jeweils bezogen auf das Gesamtgewicht der Zusammensetzung
(i) zwischen 25 und 80 Gew.-% einer Gesamtmenge von mindestens einer aromatischen Verbindung mit mindestens zwei Hydroxylgruppen, bevorzugt gemäß obenstehender allgemeiner Formel (I), ganz besonders bevorzugt von Bisphenol-A,
(ii) mehr als 1 einer Gesamtmenge von mindestens einer aromatischen Verbindung mit genau einer Hydroxylgruppe, bevorzugt von Phenol,
(iii) mehr als 1,5 Gew.-% einer Gesamtmenge von mindestens einer aromatischen Verbindung mit mindestens einem Vinylsubstituenten, bevorzugt von Styrol oder α-Methylstyrol,
wobei die Gewichtsanteile innerhalb der Gewichtsanteilbereiche (i) bis (iii) derart ausgewählt werden, dass die Summe der gewählten Gewichtanteile aus (i) bis (iii) gemeinsam mit den Gewichtsanteilen weiterer Inhaltsstoffe 100 Gew.-% ergibt.

Es ist bevorzugt, wenn die Zusammensetzung eine geringe Menge an hochsiedenden Bestandteilen gemäß Merkmal (i) enthält. Eine bevorzugte Ausführungsform der obigen Zusammensetzung enthält daher zwischen 0,5 und 10,0 Gew.-% einer Gesamtmenge von mindestens einer organischen Verbindung ohne Hydroxylgruppe mit einem Siedepunkt bei 1013 mbar von mindestens 300°C.

Als ebenfalls bevorzugte Ausführungsform enthält die Zusammensetzung zwischen 25 und 80 Gew.-% einer Gesamtmenge von aromatischen Verbindungen mit mindestens zwei Hydroxylgruppen, ausgewählt aus 4,4'-Dihydroxydiphenyl, Bisphenol-A, 2,4-Bis(4-hydroxyphenyl)-2-methylbutan, 1,1-Bis-(4-hydroxyphenyl)-cyclohexan, 1,1-Bis-(4-hydroxyphenyl)-3.3.5-trimethylcyclohexan, 4,4'-Dihydroxydiphenylsulfid, 4,4'-Dihydroxydiphenylsulfon sowie deren di- und tetrabromierten oder chlorierten Derivate wie beispielsweise 2,2-Bis(3-Chlor-4-hydroxyphenyl)-propan, 2,2-Bis-(3,5-dichlor-4-hydroxyphenyl)-propan oder 2,2-Bis-(3,5-dibrom-4-hydroxyphenyl)-propan, oder Mischungen davon.

Desweiteren ist es zusätzlich bevorzugt, wenn die vorgenannten Merkmale der vorgenannten Ausführungsformen der Merkmale (i) und (ii) der Zusammensetzung kombiniert werden.

Zusammenfassend aber nicht beschränkend gelten folgende Aspekte 1 bis 28 der Erfindung als weitere Ausführungsformen:
1. Verfahren zur Pyrolyse, umfassend mindestens die folgenden Schritte
   (a) Einbringung von Pyrolysegut, mindestens umfassend Material, enthaltend eine Mischung von Polycarbonat enthaltender Verbindung und Polystyrol enthaltender Verbindung, in einen Reaktor;
   (b) Zersetzung zumindest des in Schritt (a) eingebrachten Materials des Pyrolyseguts in dem Reaktor bei einer Temperatur von 300°C bis 700°C unter Erhalt von in der Gasphase befindlichem Produkt als Pyrolysat und von nicht in der Gasphase befindlichem Pyrolyserückstand, wobei
      (i) während der besagten Zersetzung die Menge an Sauerstoffgas im Reaktor höchstens 2,0 Vol.% bezogen auf das Gesamtvolumen der im Reaktor befindlichen Gase beträgt, und
      (ii) während der besagten Zersetzung das Pyrolysat aus dem Reaktor herausgeführt wird, und
      (iii) der besagte Pyrolyserückstand aus dem Reaktor herausgeführt wird;
   (c) Abkühlung des herausgeführten Pyrolysates auf eine Temperatur von weniger als 300°C unter Erhalt von Pyrolyseprodukt, ausgewählt aus Pyrolysatkondensat, Pyrolysatsublimat oder einer Mischung daraus;
   (d) optional Aufarbeitung des Pyrolyseprodukts.
2. Verfahren nach Aspekt 1, dadurch gekennzeichnet, dass der Reaktor ausgewählt wird aus kontinuierlichem Rührkesselreaktor (CSTR), Festbettreaktor, Fließbettreaktor, Schneckenreaktor, Schneckenförderer Reaktor, Flugstromreaktor, Mitreißstromreaktor, Drehrohrreaktor, Fließbettreaktor, Schaufelreaktor, insbesondere ausgewählt wird aus kontinuierlichem Rührkesselreaktor (CSTR), Festbettreaktor (insbesondere mit kontinuierlichem Bettaustausch (Schachtreaktor) mit innenliegenden Wärmetauscherrohren), ein Schneckenreaktor, ein Schneckenfördererreaktor, ein Flugstromreaktor, Drehrohrreaktor oder Wirbelschichtreaktor.
3. Verfahren nach einem der Aspekte 1 oder 2, dadurch gekennzeichnet, dass die Temperatur im Schritt (b) von 350°C bis 650°C, bevorzugt zwischen 400°C und 650°C, besonders bevorzugt von 420°C bis 600°C, ganz besonders bevorzugt von 450°C bis 580°C beträgt.
4. Verfahren nach einem der vorangehenden Aspekte, dadurch gekennzeichnet, dass das eingebrachte Pyrolysegut auf 300°C bis 700°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes1 Sekunde bis 2 Stunden, bevorzugt zwischen 2 Minuten und 60 Minuten, beträgt.
5. Verfahren nach einem der vorangehenden Aspekte, dadurch gekennzeichnet, dass die Herausführung des Pyrolysates aus dem Reaktor durch einen durch den Reaktor geführten Gasstrom oder durch Sog gewährleistet wird und bevorzugt dadurch eine Verweildauer des Pyrolysates als Zeitraum zwischen dem Zeitpunkt der Einbringung des besagten im Schritt (a) in den Reaktor eingebrachten Materials und dem Zeitpunkt der Ausbringung des resultierenden Pyrolysates 0,1 Sekunden bis 10 Sekunden, bevorzugt zwischen 0,5 Sekunden und 5 Sekunden, besonders bevorzugt 0,5 Sekunden bis 2 Sekunden, beträgt.
6. Verfahren nach einem der vorangehenden Aspekte, dadurch gekennzeichnet, dass die Herausführung des Pyrolysates aus dem Reaktor durch einen durch den Reaktor geführten Gasstrom gewährleistet wird, dessen Fließgeschwindigkeit im Reaktor als Leerrohrgeschwindigkeit im Bereich von 0,01 m/s bis 20 m/s liegt.
7. Verfahren nach einem der vorangehenden Aspekte, dadurch gekennzeichnet, dass das Pyrolysat im Reaktor in der Gasphase vorliegt.
8. Verfahren nach einem der vorangehenden Aspekte, dadurch gekennzeichnet, dass der Pyrolyserückstand im Reaktor festförmig ist.
9. Verfahren nach einem der vorangehenden Aspekte, dadurch gekennzeichnet, dass zumindest die Schritte (a) und (b) im Rahmen einer kontinuierlichen Prozessführung gleichzeitig ablaufen.
10. Verfahren nach einem der vorangehenden Aspekte, dadurch gekennzeichnet, dass im Schritt (b) die Menge an Sauerstoffgas im Reaktor höchstens 0,5 Vol.%, bevorzugt 0,1 Vol.%, jeweils bezogen auf das Gesamtvolumen der im Reaktor befindlichen Gase beträgt.
11. Verfahren nach einem der vorangehenden Aspekte, dadurch gekennzeichnet, dass der mit besagten Material befüllte Reaktor mit Inertgas, insbesondere mit Stickstoff, Argon, CO₂, NO oder Mischungen daraus befüllt wird.
12. Verfahren nach Aspekt 11, dadurch gekennzeichnet, dass dem Inertgas von Sauerstoffgas verschiedene reaktive Gase beigemischt werden, insbesondere ausgewählt aus Methan, gasförmigem H₂O, Wasserstoffgas oder Gemischen daraus.
13. Verfahren nach einem der vorangehenden Aspekte, dadurch gekennzeichnet, dass die Polycarbonat enthaltende Verbindung mindestens eine Verbindung ist, die mindestens zehn Struktureinheiten 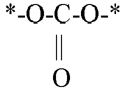 enthält, worin * eine Valenz des Polymerrückgrats kennzeichnet.
14. Verfahren nach einem der vorangehenden Aspekte, dadurch gekennzeichnet, dass die Polystyrol enthaltende Verbindung mindestens ein Polymer ist, das mindestens zehn Wiederholungseinheiten der Formel

   *-CR¹Ph-CH₂-*

   enthält, worin * eine Valenz der Wiederholungseinheit des Polymerrückgrats kennzeichnet, R¹ für ein Wasserstoffatom oder eine Methylgruppe steht und Ph eine, gegebenenfalls mit mindestens einer Gruppe ausgewählt aus (C₁ bis C₄)-Alkylgruppe, Halogenatom (insbesondere Chlor) substituierte, Phenylgruppe bedeutet.
15. Verfahren nach einem der vorangehenden Aspekte, dadurch gekennzeichnet, dass die Polycarbonat enthaltende Verbindung mindestens eine Verbindung ist, die durch Umsetzung von zumindest den Verbindungen (i) mindestens einer aromatischen Verbindung mit mindestens zwei Hydroxylgruppen, bevorzugt Bisphenol-A, und (ii) Phosgen oder Diphenylcarbonat erhalten wurde.
16. Verfahren nach einem der vorangehenden Aspekte, dadurch gekennzeichnet, dass besagtes Material in Form festförmiger Teilchen, insbesondere in Form eines körnigen Gemenges, in den Reaktor eingebracht wird.
17. Verfahren nach Aspekt 16, dadurch gekennzeichnet, dass die festförmigen Teilchen, insbesondere die losen, festförmigen Partikel des körnige Gemenges, des in den Reaktor eingebrachten besagten Materials eine einen mittleren Durchmesser X_{50,3} (Volumenmittel) von 0,01mm bis 5 cm aufweisen.
18. Verfahren nach einem der vorangehenden Aspekte, dadurch gekennzeichnet, dass besagtes Pyrolysegut im Schritt (a) neben besagtem Material zusätzlich mindestens einen Füllstoff enthält.
19. Verfahren nach Aspekt 18, dadurch gekennzeichnet, dass der Füllstoff mindestens ein Metalloxid ist, das im Rahmen der Pyrolyse von Polycarbonat nicht katalytisch wirksam ist, welches bevorzugt ausgewählt aus SiO₂.
20. Verfahren nach einem der vorangehenden Aspekte, dadurch gekennzeichnet, dass besagtes Pyrolysegut im Schritt (a) neben besagtem Material zusätzlich mindestens einen, die Reaktion der Zersetzung des besagten Materials beeinflussenden Katalysator enthält.
21. Verfahren nach Aspekt 20, dadurch gekennzeichnet, dass der Katalysator mindestens eine Verbindung ist, aus der Gruppe, die gebildet wird aus anorganischen Salzen, Mineralien, Metalloxiden, Mischoxiden, Tonen, Zeolithen.
22. Verfahren nach einem der Aspekte 20 oder 21, dadurch gekennzeichnet, dass der Katalysator ein basischer Katalysator ist.
23. Verfahren nach einem der Aspekte 20 bis 22, dadurch gekennzeichnet, dass der Katalysator ein heterogener Katalysator ist.
24. Verfahren nach einem der Aspekte 1 bis 23, dadurch gekennzeichnet, dass besagtes Pyrolysegut bezogen auf dessen Gesamtgewicht besagtes Material in einer Gesamtmenge von 10,0 bis 80,0 Gew.-%, weiter bevorzugt 30,0 bis 70,0 Gew.-%, enthält.
25. Pyrolysevorrichtung für die Erzeugung von Pyrolysat aus Pyrolysegut, enthaltend mindestens eine Dosiervorrichtung zur Einspeisung von Pyrolysegut, mindestens einen heizbaren Reaktor für die Pyrolyse, und mindestens einen Pyrolysat-Kollektor, dadurch gekennzeichnet, dass
   besagter heizbarer Reaktor für die Pyrolyse mindestens ein Heizelement enthält, das zur Temperierung des Reaktors auf eine Temperatur von 300°C bis 700°C verwendet werden kann, und mindestens einen Einlass für Pyrolysegut enthält und mindestens einen davon verschiedenen Auslass für Pyrolysat enthält; und
   Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
   heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
   mindestens ein Pyrolysat-Kollektor mindestens eine auf eine Temperatur unter 300°C temperierte Kühlvorrichtung enthält, die im besagten Kollektor zur Senkung der Temperatur des aus dem besagten Reaktor herausgeführten Pyrolysates auf weniger als 300°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält; und
   mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können.
26. Verwendung einer Pyrolyse-Vorrichtung gemäß Aspekt 25 zur Rückgewinnung von bei der Herstellung von Polycarbonat enthaltenden Verbindungen eingesetzter, organischer Verbindung mit mindestens zwei Hydroxylgruppen bei gleichzeitiger Rückgewinnung von bei der Herstellung von Polystyrol-haltiger Verbindung verwendetem Styrol, durch simultaner Pyrolyse besagter Polycarbonat enthaltender Verbindung und besagter Polystyrol-haltigen Verbindung.
27. Verwendung nach Aspekt 26, dadurch gekennzeichnet, dass die Pyrolyse-Vorrichtung zur Rückgewinnung von bei der Herstellung von Polycarbonat enthaltender Verbindung eingesetztem Bisphenol-A verwendet wird.
28. Zusammensetzung, mindestens enthaltend jeweils bezogen auf das Gesamtgewicht der Zusammensetzung
   (i) zwischen 25 und 80 Gew.-% einer Gesamtmenge von mindestens einer aromatischen Verbindung mit mindestens zwei Hydroxylgruppen, bevorzugt von Bisphenol-A,
   (ii) mehr als 1 Gew.-% einer Gesamtmenge von mindestens einer aromatischen Verbindung mit genau einer Hydroxylgruppe, bevorzugt von Phenol,
   (iii) mehr als 1,5 Gew.-% einer Gesamtmenge von mindestens einer aromatischen Verbindung mit mindestens einem Vinylsubstituenten, bevorzugt von Styrol oder α-Methylstyrol,
   wobei die Gewichtsanteile innerhalb der Gewichtsanteilbereiche aus (i) bis (iii) derart ausgewählt werden, dass die Summe der gewählten Gewichtanteile aus (i) bis (iii) gemeinsam mit den Gewichtsanteilen weiterer Inhaltsstoffe 100 Gew.-% ergibt.

### Beispiele

### Beispiel 1 (erfindungsgemäß):

Die Pyrolyse von Polycarbonat ABS Blend FC 85 von Covestro wurde in einem mit N₂ durchströmten Festbettreaktor bei 500 °C durchgeführt. Das Polycarbonat wurde in 5 Al₂O₃ Tiegeln mit Halterung mit jeweils 2 g in den Reaktor eingebracht. Die Verweilzeit des Polycarbonat Blends betrugt 30 min. Die Fließgeschwindigkeit des Gasstroms (Leerrohrgeschwindigkeit) im Reaktor betrug 0,07 m/s. Hinter dem Reaktor befanden sich 2 Kondensatoren, um die Flüssigkomponenten aus dem entstehenden Pyrolyseprodukt abzutrennen und aufzusammeln. Der entstehende Koksanteil des Pyrolyserückstandes wurde durch Wiegen der Tiegel nach der Pyrolyse bestimmt. Das Gas nach den 2 Kondensatoren wurde mittels GC charakterisiert. Die Komponenten im als Öl anfallendem Pyrolyseprodukt wurden mit GC-FID bestimmt. Dazu wurde ein Agilent 7890A mit einer Säule SupelcoSPB 50 benutzt. Das Pyrolyse Öl wurde mit Aceton 1:50 oder 1:100 verdünnt.

### Beispiel 2 (nicht erfindungsgemäß):

Die Pyrolyse von Polycarbonat ABS Blend FC 85 von Covestro wurde in einem mit N₂ durchströmten Festbettreaktor bei 800 °C durchgeführt. Das PC wurde in 5 Al₂O₃ Tiegeln mit Halterung mit jeweils 2 g in den Reaktor eingebracht. Die Verweilzeit des PC Blends betrug 30 min . Die Fließgeschwindigkeit des Gasstroms (Leerrohrgeschwindigkeit) im Reaktor betrug 0,07 m/s. Hinter dem Reaktor befanden sich 2 Kondensatoren, um die Flüssigkomponenten aus dem entstehenden Pyrolyseprodukt abzutrennen und aufzusammeln. Der entstehende Koksanteil des Pyrolyserückstandes wurde durch Wiegen der Tiegel nach der Pyrolyse bestimmt. Das Gas nach den 2 Kondensatoren wurde mittels GC charakterisiert. Die Komponenten im als Öl anfallendem Pyrolyseprodukt wurden mit GC-FID bestimmt.

Ergebnis als Anteile bezogen auf Gewichtsteile eingesetztes PC-Blend:

| **Pyrolyse Temperatur** | **Gas** | **Koks** | **weitere Bestandteile*** | **Toluol*** | **Ethylbenzol*** | **Styrol*** | **Phenol*** | **4-tert-ButylPhenol*** | **Phthalsäure*** | **BPA*** |
|---|---|---|---|---|---|---|---|---|---|---|
| 500°C (Bsp.1) | 7,5 | 10,8 | 23,3 | 0,3 | 0,5 | 7,8 | 11,9 | 0,2 | 0,3 | 34,5 |
| 800°C (Bsp.2) | 9,4 | 7,9 | 19,8 | 0,5 | 0,8 | 11,5 | 20,7 | 0,3 | 0,1 | 18,2 |

| | | | | | | | | | | |
|---|---|---|---|---|---|---|---|---|---|---|
| * kennzeichnet Bestandteile des Pyrolyseproduktes | | | | | | | | | | |

## Patentansprüche

1. Verfahren zur Pyrolyse, umfassend mindestens die folgenden Schritte
(a) Einbringung von Pyrolysegut, mindestens umfassend Material, enthaltend eine Mischung von Polycarbonat enthaltender Verbindung und Polystyrol enthaltender Verbindung, in einen Reaktor;
(b) Zersetzung zumindest des in Schritt (a) eingebrachten Materials des Pyrolyseguts in dem Reaktor bei einer Temperatur von 300°C bis 700°C unter Erhalt von in der Gasphase befindlichem Produkt als Pyrolysat und von nicht in der Gasphase befindlichem Pyrolyserückstand, wobei
(i) während der besagten Zersetzung die Menge an Sauerstoffgas im Reaktor höchstens 2,0 Vol.% bezogen auf das Gesamtvolumen der im Reaktor befindlichen Gase beträgt, und
(ii) während der besagten Zersetzung das Pyrolysat aus dem Reaktor herausgeführt wird, und
(iii) der besagte Pyrolyserückstand aus dem Reaktor herausgeführt wird;
(c) Abkühlung des herausgeführten Pyrolysates auf eine Temperatur von weniger als 300°C unter Erhalt von Pyrolyseprodukt, ausgewählt aus Pyrolysatkondensat, Pyrolysatresublimat oder einer Mischung daraus;
(d) optional Aufarbeitung des Pyrolyseprodukts.

2. Verfahren nach Anspruch 1, **dadurch gekennzeichnet, dass** die Temperatur im Schritt (b) von 350°C bis 650°C, bevorzugt zwischen 400°C und 650°C, besonders bevorzugt von 420°C bis 600°C, ganz besonders bevorzugt von 450°C bis 580°C beträgt.

3. Verfahren nach einem der vorangehenden Ansprüche, **dadurch gekennzeichnet, dass** das eingebrachte Pyrolysegut auf 300°C bis 700°C temperiert wird und nach Erreichen dieser Zieltemperatur die Verweildauer des entsprechend temperierten Pyrolysegutes bis zum Zeitpunkt der Ausbringung seines resultierenden Pyrolyserückstandes 1 Sekunde bis 2 Stunden, bevorzugt zwischen 2 Minuten und 60 Minuten, beträgt.

4. Verfahren nach einem der vorangehenden Ansprüche, **dadurch gekennzeichnet, dass** die Herausführung des Pyrolysates aus dem Reaktor durch einen durch den Reaktor geführten Gasstrom oder durch Sog gewährleistet wird und bevorzugt dadurch eine Verweildauer des Pyrolysates als Zeitraum zwischen dem Zeitpunkt der Einbringung des besagten im Schritt (a) in den Reaktor eingebrachten Materials und dem Zeitpunkt der Ausbringung des resultierenden Pyrolysates 0,1 Sekunden bis 10 Sekunden, bevorzugt zwischen 0,5 Sekunden und 5 Sekunden, besonders bevorzugt 0,5 Sekunden bis 2 Sekunden, beträgt.

5. Verfahren nach einem der vorangehenden Ansprüche, **dadurch gekennzeichnet, dass** die Herausführung des Pyrolysates aus dem Reaktor durch einen durch den Reaktor geführten Gasstrom gewährleistet wird, dessen Fließgeschwindigkeit im Reaktor als Leerrohrgeschwindigkeit im Bereich von 0,01 m/s bis 20 m/s liegt.

6. Verfahren nach einem der vorangehenden Ansprüche, **dadurch gekennzeichnet, dass** zumindest die Schritte (a) und (b) im Rahmen einer kontinuierlichen Prozessführung gleichzeitig ablaufen.

7. Verfahren nach einem der vorangehenden Ansprüche, **dadurch gekennzeichnet, dass** im Schritt (b) die Menge an Sauerstoffgas im Reaktor höchstens 0,5 Vol.%, bevorzugt 0,1 Vol.%, jeweils bezogen auf das Gesamtvolumen der im Reaktor befindlichen Gase beträgt.

8. Verfahren nach einem der vorangehenden Ansprüche, **dadurch gekennzeichnet, dass** der mit besagten Material befüllte Reaktor mit Inertgas, insbesondere mit Stickstoff, Argon, CO₂, NO oder Mischungen daraus befüllt wird.

9. Verfahren nach einem der vorangehenden Ansprüche, **dadurch gekennzeichnet, dass** die Polycarbonat enthaltende Verbindung mindestens eine Verbindung ist, die mindestens zehn Struktureinheiten 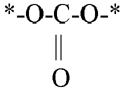 enthält, worin * eine Valenz des Polymerrückgrats kennzeichnet.

10. Verfahren nach einem der vorangehenden Ansprüche, **dadurch gekennzeichnet, dass** die Polystyrol enthaltende Verbindung mindestens ein Polymer ist, das mindestens zehn Wiederholungseinheiten der Formel
*-CR¹Ph-CH₂-*
enthält, worin * eine Valenz der Wiederholungseinheit des Polymerrückgrats kennzeichnet, R¹ für ein Wasserstoffatom oder eine Methylgruppe steht und Ph eine, gegebenenfalls mit mindestens einer Gruppe ausgewählt aus (C₁ bis C₄)-Alkylgruppe, Halogenatom (insbesondere Chlor) substituierte, Phenylgruppe bedeutet.

11. Verfahren nach einem der vorangehenden Ansprüche, **dadurch gekennzeichnet, dass** die Polycarbonat enthaltende Verbindung mindestens eine Verbindung ist, die durch Umsetzung von zumindest den Verbindungen (i) mindestens einer aromatischen Verbindung mit mindestens zwei Hydroxylgruppen, bevorzugt Bisphenol-A, und (ii) Phosgen oder Diphenylcarbonat erhalten wurde.

12. Verfahren nach einem der vorangehenden Ansprüche, **dadurch gekennzeichnet, dass** besagtes Material in Form festförmiger Teilchen, insbesondere in Form eines körnigen Gemenges, in den Reaktor eingebracht wird.

13. Verfahren nach einem der vorangehenden Ansprüche, **dadurch gekennzeichnet, dass** besagtes Pyrolysegut im Schritt (a) neben besagtem Material zusätzlich mindestens einen Füllstoff enthält.

14. Verfahren nach einem der Ansprüche 1 bis 13, **dadurch gekennzeichnet, dass** besagtes Pyrolysegut bezogen auf dessen Gesamtgewicht besagtes Material in einer Gesamtmenge von 10,0 bis 80,0 Gew.-%, weiter bevorzugt 30,0 bis 70,0 Gew.-%, enthält.

15. Pyrolysevorrichtung für die Erzeugung von Pyrolysat aus Pyrolysegut, enthaltend mindestens eine Dosiervorrichtung zur Einspeisung von Pyrolysegut, mindestens einen heizbaren Reaktor für die Pyrolyse, und mindestens einen Pyrolysat-Kollektor, **dadurch gekennzeichnet, dass**
besagter heizbarer Reaktor für die Pyrolyse mindestens ein Heizelement enthält, das zur Temperierung des Reaktors auf eine Temperatur von 300°C bis 700°C verwendet werden kann, und mindestens einen Einlass für Pyrolysegut enthält und mindestens einen davon verschiedenen Auslass für Pyrolysat enthält; und
Dosiervorrichtung und heizbarer Reaktor für die Pyrolyse derart zueinander angeordnet und ausgestaltet sind, dass die Dosiervorrichtung über mindestens eine Zuleitung mit einem Einlass für Pyrolysegut des besagten Reaktors in Verbindung steht; und
heizbarer Reaktor für die Pyrolyse und Pyrolysat-Kollektor miteinander in Fluidverbindung stehen, so dass die Herausführung des, bevorzugt gasförmigen, Pyrolysats aus besagtem Auslass für Pyrolysat und die Einbringung des herausgeführten Pyrolysats in den Pyrolysat-Kollektor möglich ist; und
mindestens ein Pyrolysat-Kollektor mindestens eine auf eine Temperatur unter 300°C temperierte Kühlvorrichtung enthält, die im besagten Kollektor zur Senkung der Temperatur des aus dem besagten Reaktor herausgeführten Pyrolysates auf weniger als 300°C unter Bildung von Pyrolyseprodukt, ausgewählt aus Pyrolysatkondensat, Pyrolysatsublimat oder einer Mischung daraus, verwendbar ist, und mindestens einen Behälter zur Sammlung und Ausbringung des unter Kühlung erhaltenen Pyrolyseprodukts enthält; und
mindestens eine Dosiervorrichtung zur Einspeisung von Pyrolysegut, mindestens ein heizbarer Reaktor für die Pyrolyse, und mindestens ein Pyrolysat-Kollektor derart zueinander angeordnet und ausgestaltet sind, dass sie gleichzeitig betrieben werden können.

16. Verwendung einer Pyrolyse-Vorrichtung gemäß Anspruch 15 zur Rückgewinnung von bei der Herstellung von Polycarbonat enthaltenden Verbindungen eingesetzter, organischer Verbindung mit mindestens zwei Hydroxylgruppen bei gleichzeitiger Rückgewinnung von bei der Herstellung von Polystyrol-haltiger Verbindung verwendetem Styrol, durch simultaner Pyrolyse besagter Polycarbonat enthaltender Verbindung und besagter Polystyrol-haltigen Verbindung.

17. Zusammensetzung, mindestens enthaltend jeweils bezogen auf das Gesamtgewicht der Zusammensetzung
(i) zwischen 25 und 80 Gew.-% einer Gesamtmenge von mindestens einer aromatischen Verbindung mit mindestens zwei Hydroxylgruppen, bevorzugt von Bisphenol-A,
(ii) mehr als 1 Gew.-% einer Gesamtmenge von mindestens einer aromatischen Verbindung mit genau einer Hydroxylgruppe, bevorzugt von Phenol,
(iii) mehr als 1,5 Gew.-% einer Gesamtmenge von mindestens einer aromatischen Verbindung mit mindestens einem Vinylsubstituenten, bevorzugt von Styrol oder α-Methylstyrol,
wobei die Gewichtsanteile innerhalb der Gewichtsanteilbereiche aus (i) bis (iii) derart ausgewählt werden, dass die Summe der gewählten Gewichtanteile aus (i) bis (iii) gemeinsam mit den Gewichtsanteilen weiterer Inhaltsstoffe 100 Gew.-% ergibt.
